# EUROPEAN PATENT SPECIFICATION

(11) **EP 1 324 783 B1**
(45) Date of publication and mention of the grant of the patent: **22.03.2006**
(21) Application number: 01983460.5
(22) Date of filing: 31.08.2001
(51) Int. Cl.: A61L 27/50, A61L 26/00

(54) **AREAL IMPLANT WITH ULTRASONICALLY DETECTABLE ELEMENTS**
FLÄCHIGES IMPLANTAT MIT ULTRASCHALL-SICHTBAREN ELEMENTEN
IMPLANT ZONAL D'ELEMENTS DETECTABLES PAR ULTRASONS

(30) Priority: 11.10.2000 DE 10050199
(43) Date of publication of application: 09.07.2003
(73) Proprietor: Ethicon GmbH, 22851 Norderstedt (DE)
(72) Inventor: PRIEWE, Jörg, 24114 Kiel (DE); SCHULDT-HEMPE, Barbara, 24576 Bad Bramstedt (DE); WALTHER, Christoph, 24568 Kattendorf (DE)
(74) Representative: UEXKÜLL & STOLBERG
(86) International application number: PCT/EP2001/010086
(87) International publication number: WO 2002/030482

(56) References cited:
- WO-A-01/56499
- US-A- 4 636 213
- US-A- 5 201 314
- US-A- 5 320 100
- US-A- 5 487 390

## Description

The invention relates to an areal implant with a flexible basic structure on a polymer basis.

Areal implants with a flexible basic structure on a polymer basis, which are manufactured for example in the form of meshes or tapes, are widespread. They are used for example in a surgical procedure in order to support or strengthen an organ or tissue or to promote the healing process. Often, such an implant must remain permanently or at least for some time in a patient's body. In this case, the basic structure contains non-absorbable polymer or slowly-absorbable polymer.

Over time, the inserted implant can shift, shrink or fold. This can cause the patient problems. This can be diagnostically recorded only with difficulty if at all with imaging processes, as conventional areal implants are relatively fine, in order to guarantee a sufficient flexibility, and only a short time after the procedure tissue has grown through them to the extent that they can hardly, if at all, be recognized any longer using customary and widespread diagnostic methods such as ultrasound or x-rays so that no diagnostically usable pronouncements are possible.

Thus, after the implantation of thin areal polymer meshes, (e.g. for repairing inguinal or abdominal hernias) or tapes (which are used e.g. in the bladder area), the implants are, admittedly, initially shown well in the ultrasound as they are surrounded by a liquid echo-poor border (seroma). However, the contrast subsequently lessens (see also H.F. Weiser and M. Birth, Vizeral-chirurgische Sonographie, p. 315-316, Springer Verlag 2000). This can result in the cause of problems being only insufficiently recognized and a subsequent handling of the implant being no longer possible, as this is only insufficiently, or not at all, detectable with customary equipment.

In WO 98/19713, coating processes for medical devices (such as e.g. catheters or syringes) are described which produce echogenic structures, i.e. those detectable in the ultrasound, on the surface. The contrast in the ultrasound image is achieved by boundary surfaces between gas and dense media. The proposed coatings are however not suitable for use with long-term implants. Thus polyurethane coatings are sensitive to hydrolysis and have toxic residual monomers (diisocyanates) and decomposition products. There are many items in the literature which refer to the critical properties of diisocyanates and the prepolymers prepared from them (e.g. Zissu et al., Contact Dermatitis 39(5), 248-251 (Nov. 1998)), but also of the decomposition products, such as aromatic diamines (e.g. Batich et al., J. Biomed. Mater. Res. 23 (A3 Suppl), 311-319 (Dec. 1989)). These are discussed as the cause of delayed pain and allergic reactions after implantation of polyurethanes. A further problem in the case of the coatings disclosed in WO 98/19713 is the mechanical stability on the implant. Precisely in the case of the smooth polymers often used in implant meshes, such as polypropylene, polytetrafluorethylene and polyvinylidene fluoride, a simple dipping process can lead to a defective adhesion on the implant; the thin echogenic film would crumble over time, in particular on bending. The polyacrylic acid coatings also described produce, through an entry of gas bubbles into an aqueous solution of polyacrylic acid, a foam which is deposited on the medical apparatus. As these acrylates are soluble in water, it is to be assumed that this formulation does not lead to a lasting echo contrast as is required for long-term implants. Furthermore, it is mentioned that this coating contains channels which are however open-pored and therefore fill up relatively easily and lose their contrast thereby. In addition, crater-shaped indentations are disclosed which can at best however evoke a brief signal amplification as these indentations are wetted over time and gas bubbles situated in them dissolve.

In the case of commercially available ultrasound contrast agents such as e.g. "Albunex" (trade name of Molecular Biosystems, Inc.), the defective pressure stability is problematic. Even low physiologically-occurring pressures (Vuille et al., J. Am. Soc. Echocardiogr. 7(4), 347-354 (July-Aug. 1994); A. Braymann, J. Acoust. Soc. Am. 99(4Pt1), 2403-2408 (1996)) or too great a pressure, such as can occur in the case of too-rapid an injection or a small cannula, can damage the contrast agent so greatly (Sonne et al., Int. J. Cardiac Imaging 11(1), 47-53 (1995)) that only little or no activity remains. Gottlieb et al. (J. Ultrasound in Medicine, 14(2), 109-116 (1995)) observed in a videodensiometric in-vitro model a pressure dependency of the destruction of "Albunex" at physiological pressures of 10 - 180 mm Hg and point to the need for an ultrasound contrast agent sufficiently stable at physiological pressures.

Therefore, ultrasound contrast agents such as "Albunex" are not suitable for use as long-term implant meshes despite the proposal in WO 98/19713 to use "Albunex" as gas-containing starting material for echogenic coatings. Because of the high pressure sensitivity, even a slight coughing of the implant carrier could destroy the echogenicity of the implant. There is also an enzymatic sensitivity.

In WO 95/01165, physiologically acceptable organic aerogels and pyrolyzed aerogels (i.e. carbon aerogels) for medical purposes are described. However, due to the materials, none of the embodiments appears suitable for use with a long-term implant. Thus the adducts mentioned made from resorcin, melamine or resorcinol with formaldehyde as well as the carbon aerogels are not customary implant materials. Furthermore, no suitable sealing is disclosed which prevents such an aerogel, when used as ultrasound contrast agent from quickly losing its gas content after an implantation, nor are there references to a coating on, or attachment to flat, flexible polymer implants.

US 5,081,997 describes a number of possibilities of arranging sound-reflecting materials, such as e.g. glass particles with a diameter of 5 *µ*m, on medical products such as e.g. a catheter. Hollow particles are also mentioned. In addition to these sound-reflecting materials, gases can be contained in a matrix. However, there are no references to uses with areal long-term implants.

In US 5,327,891, it is shown how the detectability of a catheter in the ultrasound can be improved using microbubbles.

WO 00/09178 discloses composites made of plastic and particularly heavy nanoparticles (density at least 5 g/cm³) which improve the detectability of a medical device (e.g. a biopsy needle) in the ultrasound. For a use with areal long-term implants, however, such relatively heavy particles are less suitable.

In recent years, there have been numerous approaches to the manufacture of ultrasound contrast agents for intravenous use. This essentially involves stabilized microbubbles which are produced e.g. by shaking porous sugar microparticles ("Echovist", Schering AG) which can also contain a fatty acid ("Levovist", Schering AG; Chapter 7 in B.B. Goldberg, "Ultrasound Contrast Agents", Martin Dunits Ltd, 1997), or slightly cross-linked, gas-filled protein microcapsules ("Albunex", Molecular Biosystems, Inc.; "Optison", MBI). There are also numerous approaches to the manufacture of gas-filled resorbable polymer microparticles which are manufactured on the basis of polyactides, polycaprolactones and other resorbable polymers.

However, none of the known products is capable in itself of producing a lasting ultrasound contrast over a prolonged period, as the stabilizing bubbles either dissolve in the blood or tissue or the protein or polymer shell decomposes as a result of simple hydrolysis or enzymatic splitting. Thus, polymer microparticles made from polybutylcyanoacrylates mentioned e.g. in EP 0 644 777 B1 are decomposed so rapidly in serum that after 4 hours the previously cloudy suspension is completely clear and a metabolite is to be 100% detected. Such particles in this form are not suitable for use with long-term implants.

Another problem is the preparation processes for the microcapsules which are based for the most part on oil-in-water processes or water-in-oil processes. In this case, a gas core must be produced e.g. by freeze-drying, for which a not completely impervious wall is required. However, water can also enter again through this slightly porous wall; through the gas loss associated with this, the ultrasound contrast decreases.

WO 01/56499 discloses an areal implant with a flexible basic structure on a polymer basis and with X-ray-detectable elements which can be detected for a long period after implantation. Generally, X-ray-detectable elements are detectable by ultrasonic diagnostics equipment as well. In order to achieve a high X-ray contrast, however, the X-ray-detectable elements have to include chemical elements having a high atomic number which are generally not required for ultrasonic detectability purposes.

The object of the invention is to provide an alternative areal implant with a flexible basic structure on a polymer basis which, after implantation in a patient, can for some time or permanently be detected reliably with diagnostic ultrasound processes.

This object is achieved by an areal implant with the features of claim 1. Advantageous versions of the invention emerge from the dependent claims. Claims 30 to 55 relate to processes for producing such implants.

The areal implant according to the invention has a polymer-based flexible basic structure and ultrasonically detectable elements. These elements contain or produce gas. By a gas-producing element is meant an element which releases a gas after insertion of the implant in a patient's body or during an ultrasound examination, e.g. due to the temperature within the patient being higher compared with room temperature or due to the ultrasound field. The gas-containing character of the elements detectable with ultrasound, which is therefore present at least during an ultrasound examination, effects a good contrast in the ultrasound image, for which reason the implant according to the invention can reliably be made visible with an ultrasound process. The elements detectable with ultrasound are set up to be detectable for at least four weeks after implantation so that the implant can be detected even some time after the procedure or even permanently. As is described below in detail, there are various possibilities for such long-term-stable echogenic elements. Although the word "elements" is plural here, a corresponding implant which contains only one such element naturally equally forms part of the invention. In the following, instead of "detectable with ultrasound" or "ultrasonically detectable", the term "echogenic" is also used.

The implant is preferably set up for a permanent implantation, but can also be resorbable. The ultrasonically detectable elements are therefore present in histocompatible form and are biocompatible, i.e. if at all possible do not give off toxic substances even after a long time, and are preferably permanently connected to the basic structure. The implant is preferably flexible as a whole. The elements detectable with ultrasound enable the implant to be made visible as required at any time after the surgical procedure or upon insertion of the implant.

The invention enables areal, flexible long-term implants (e.g. tapes or meshes) to be made detectable in the ultrasound, the properties such as low weight, flexibility, flexural strength, elasticity or tensile strength of the implant being unchanged, or only slightly changed, vis-à-vis a conventional implant. The echogenic elements permit the implant to be recognized well with diagnostic ultrasound procedures for the time of the implantation. An unequivocal recognition of the implant is possible; it stands out sufficiently from the body's own structures, such as e.g. fasciae. Furthermore, a sufficient mechanical stability of the marking in the form of the echogenic elements and a secure attachment to the flexible basic structure of the implant are ensured.

For use as an implant, conditions such as the harmlessness of the contents and of possible decomposition products can be fulfilled. As essentially long-term implants are involved, the echogenic properties are set so that the marking in the form of the echogenic elements is matched to the respective requirement. A non-resorbable or partially resorbable implant should therefore have markings which are best to be detected for the duration of the implantation or at least for the period of time in which, experience shows, complications occur. A resorbable implant, on the other hand, should contain markings which are best visible for the period when the basic structure of the implant is present and are then quickly broken down or eliminated from the body. The decomposition profile of the echogenic elements is preferably matched, by suitable choice of material, to that of the basic structure of the implant.

The implant according to the invention is detectable with conventional, also older ultrasound equipment, but takes account of new developments in instrument technology in which e.g. particular resonance effects, non-linear effects, stimulated acoustic emission (see also Forsberg, "Physics of Ultrasound Contrast Agents", Chapter 2 in "Ultrasound Contrast Agents", B. Goldberg (ed), Martin Dunitz Ltd 1997), Harmonic Imaging, Powerdoppler, Pulse Inversion Harmonic Imaging (HDI 5000 from ATL), Siemens Ensemble Tissue Harmonic Imaging (Sonoline Elegra, Sonoline Omnia) or new trends in image processing, e.g. 3D processes or the so-called SieScape® process, are used.

The echogenic elements can be arranged so that other diagnostic procedures, such as x-ray or magnetic resonance examinations or ultrasound examinations of structures lying behind them, are not disturbed by excessive shading.

It is particularly advantageous if the ultrasonically detectable elements are arranged in an areal pattern. This is because in this case a shift of the implant or sections of the implant (e.g. a folding round a corner) can be easily recognized on the ultrasound image. Even a shrinking or stretching can be observed from the changed distances between the individual components of the pattern. Furthermore, it is possible by means of the pattern to mark particularly interesting areas of the implant for a subsequent treatment, such as cutting, injection of an auxiliary agent or tightening, under preferably minimally invasive conditions and accompanied by ultrasound monitoring. A pattern is also advantageous upon recognition of the implant if the implant (or parts thereof) is later to be removed again. Not least, the sonographic detectability of the implant during the implantation is quite generally improved by a pattern.

The basic structure can contain non-resorbable polymer, resorbable polymer or mixtures of non-resorbable and resorbable polymer. The basic structure thus preferably contains one or more implantable polymers which are optionally partially, completely or not resorbable, or mixtures of such polymers.

Examples of histocompatible non-resorbable or very slowly resorbable substances are polyalkenes (e.g. polypropylene or polyethylene), fluorinated polyolefins (e.g. polytetrafluoroethylene or polyvinylidene fluoride), polyamides, polyurethanes, polyisoprenes, polystyrenes, polysilicones, polycarbonates, polyarylether ketones (PEEKs), polymethacrylic acid esters, polyacrylic acid esters, aromatic polyesters, polyimides as well as mixtures and/or co-polymers of these substances. There can be considered as resorbable substances, for example, polyhydroxy acids (e.g. polylactides, polyglycolides, polyhydroxybutyrates, polyhydroxyvaleriates), polycaprolactones, polydioxanones, synthetic and natural oligo- and polyamino acids, polyphosphazenes, polyanhydrides, polyorthoesters, polyphosphates, polyphosphonates, polyalcohols, polysaccharides, polyethers, resorbable glasses as well as mixtures and/or co-polymers of such substances; preferably, the in vivo resorption duration is more than 30 days.

The flexible basic structure is preferably constructed as a mesh, tape, film or perforated film and can be of conventional type in principle. Preferably, it is thinner than 1 mm. It is conceivable that the shape of an implant to be used in a given operation is cut to size from a larger piece of material before the operation.

Echogenic elements which are particularly clearly visible in ultrasound procedures contain encapsulated gases or compounds which generate gas under physiological conditions and/or ultrasound. Particularly suitable are non-toxic and chemically stable elements or chemical compounds with these properties as end products.

Preferably, the echogenic elements have a structural material (i.e. a material from which the echogenic elements are essentially manufactured apart from the gas or the gas-generating substance), which corresponds to the materials of the basic structure. The echogenic elements can thus likewise be non-resorbable, partially resorbable or completely resorbable.

In the case of non-resorbable implants, biocompatible, closed-cell foams or syntactic foams in the form of linear structures (preferably threads) or pre-shaped bodies are preferably attached to the implant either subsequently or during the manufacture of the flexible basic structure. By syntactic foams are meant polymer materials the gas-filled closed cells of which are produced by hollow balls as filler in the matrix.

Through an arrangement in the form of patterns, such pre-shaped bodies or threads can be attached to the basic structure so that the implant is not, or poorly, visible in areas in the ultrasound and contains areas with good visibility. These markings permit an unequivocal recognition and differentiation of the body's own structures.

Open-cell foams should be used only in the case of syntactic foams and have an external pore diameter less than the particle size. A borderline case is hydrogels which contain gas-filled microparticles.

The materials of the threads and pre-shaped bodies are preferably foamed polyolefins for which there is no fear of hydrolytic decomposition of the main chain even in the case of long-term implantation (e.g. polypropylene, polyethylene, polyvinylidene fluoride, polytetralfuoroethylene). There are numerous processes for preparing foams, mostly from the 1960s or earlier (see also "Foamed Plastics" in Ullmann's Encyclopedia of Industrial Chemistry Vol. A11, p 435 ff, 5^{th} edition 1988) .

However, suitable metal foams, e.g. made from sintered-together thin-walled gas-filled titanium or steel microcapsules as produced at the Georgia Institute of Technology by Dr. Cochran's working group, or glass foams, can also be used.

For example gases, such as nitrogen, oxygen, CO₂, perfluoroalkanes, fluorinated alkanes, SF₆, rare gases or also alkanes or cyloalkanes which are physiologically harmless in small doses, can be incorporated into the polymer using direct gassing processes during extrusion. But this can also take place under supercritical conditions such as e.g. in the so-called MuCell™ process (Trexel Inc.). It is advantageous to use gases which have only a low permeability in the polymer and dissolve only a little in blood or plasma, e.g. perfluoroalkanes in polypropylene.

A further possibility is expansion with swelling agents (blowing agents) as described in the current literature. Toxicologically problematic substances such as azo compounds should be used only when these or their decomposition products are sufficiently encapsulated. More suitable are substances such as baking powder, water or easily decarboxylizable substances such as e.g. malonic acid and its esters.

By means of such processes, echogenic pre-shaped bodies or else threads or knitted products can be applied in different patterns to the basic structure of the implant. The advantage of a pattern-form arrangement is the distinguishability of the body's own structures.

The gases can however also be included permanently in pre-shaped bodies or threads by means of an encapsulation of hollow glass bodies (e.g. "Scotchlite", trade name of 3M, of "Q-Cel", trade name of the PQ Corp.), expanded silicates (e.g. "Perlite Hollow Spheres", trade name of The Schundler Company), glass foams or gas-filled polymer capsules (e.g. "Plastic Microspheres" of the PQ Corp), aerogels or hollow threads (e.g. "Hollofil", trade name of DuPont). The encapsulation can be carried out e.g. by means of spray-coating, solvent evaporation, compounding or extrusion.

A further possibility consists of encapsulating carbon nanopipes in a pre-shaped body or thread. Poncharal et. al. (Science 283, 1513-1516 (March 5, 1999) showed that carbon nanopipes can display a very sharp electromechanical resonance in the region of several MHz. By means of novel ultrasound analysis procedures, this resonance sharpness of the basic frequency, but also of the harmonic frequencies, should be exploitable to separate the implant very much better from the response signal of the surrounding tissue.

In particular in the case of polymers with hydrolyzable side chains, such as polyacrylic acid esters or polymethacrylic acid esters, the use of stable hollow bodies in the pre-shaped bodies or threads may be advisable as otherwise a loss of contrast through the loss of gas upon hydrolysis and expansion can result. An additional hydrolysis-stable cross-linking of the polymers may be advisable in order that the gas-filled glasses or polymer particles do not stray from a marking in a pattern.

Pre-shaped bodies can e.g. be prepared from the polymerization of methyl methacrylate in poly (methylacrylate, methyl methacrylate) reacted with hollow glass bodies with a suitable starter system (e.g. benzoyl peroxide and N,N'-dimethyl-p-toluidine). Such monomer-polymer systems have been used since the 1960s in bone cements, and are therefore also to be regarded as long-term biocompatible. To achieve good processing properties, the viscous properties can also be set with pigments, such as aerosil.

A further possibility is to encapsulate echogenic gas-filled microcapsules (e.g. ultrasound contrast agents). These should have a sufficient pressure, temperature and storage stability. The inclusion of the contrast agents can be carried out, e.g. via introduction into tubes or tubular films. It can be useful to add acids, bases or buffer systems which repress the hydrolysis of the contrast agents; furthermore, gels can prevent enzymes from approaching the contrast agents. Preferably, however, ultrasound contrast agents should be prepared which are stable over a long period of time, best of all non-resorbable. Limitations such as are essential e.g. for parenteral use, namely that the particles must be vessel-accessible and thus should have a diameter of less than 10 *µ*m, do not apply here.

The incorporation of gas-filled, echogenic structures into hydrogels also has the advantage that hydrogel objects of themselves offer a certain distinguishability of the seroma-free implant such as is present in the body after a while. These objects can appear seroma-like in the ultrasound image. Biocompatible natural and/or synthetic polymers can be considered as materials for these hydrogels, depending on application. Ionically or chemically cross-linked polyamino acids, synthetic polyelectrolytes and partially, non- or fully hydrolyzed polyacrylic, polymethacrylic or polycyanacrylic esters can be named. Furthermore, hydrogels which contain polyethylene glycols (PEGs), polyvinyl alcohols (PVAs), polyvinylpyrrolidones (PVPs) or mono-, oligo- or polysaccharides, can be named.

The position of the implant in the body can thus be established via the pattern-form arrangement and shape of such echogenic elements without the diagnosis of a genuine seroma or an inflammation wrongly being positively or negatively distorted. Thus, in addition to gas-filled objects, fluid-filled objects can also be advantageous.

The encapsulation of echogenic, gas-filled microcapsules also has the advantage that they not only generate a certain positive contrast through their back-scatter but also through size and wall thickness, the resonance frequency of this scattering can be set at the diagnostically customary range (0.5 to 20 MHz), which leads to an amplified echosignal at the excitation frequency. In addition, non-linear effects, such as e.g. in the case of harmonic imaging, can be used. Furthermore, colour-doubler effects which e.g. are called "stimulated acoustic emission" (Blomley et al., Ultrasound in Medicine and Biology 25(9), 1341-52 (November 1999)), of these particles can be used.

The echogenic microcapsules can be constructed so that they are stable in the human body for approximately four weeks to several years. Thus, echogenic microparticles e.g. of long-chained cyanoacrylates (hexyl, heptyl, octyl, nonyl,...) or methacrylic acid esters can be prepared. Mixed particles consisting of non-resorbable and resorbable polymers can also be used.

In the case of slowly resorbable polymer implants, such as some polylactides, polylactide glycolides, polycaprolactones or polydioxanones and other polyesters (polyhydroxy acids such as e.g. polyhydroxybutyric acid, polyhydroxyvaleric acid), polyether esters and polyamides and their mixtures and copolymers, the gases can be incorporated as with the non-resorbable polymers. However, non-resorbable carriers are excluded. Instead, decomposable glass capsules or resorbable echogenic polymer microcapsules are preferably used for the preparation of syntactic foams or predominantly closed-cell foams, as already described, prepared from the materials of the flexible basic structure.

As the decomposition of resorbable polymers can also depend, apart from the chemical composition and the chain length, on factors such as size, porosity and the general conditions in the tissue (e.g. substance transport), the echogenic regions should be matched in their decomposition and resorption properties to the actual implant. An influence can be exerted in addition with additional coatings with resorbable substances (such as e.g. fats, waxes, polymers, inorganic minerals), compounded polymer additives (such as e.g. oxidic, carbonated pigments, carboxylic acids, anhydrides) or compounded polymers which influence the expansion and decomposition behaviour.

In one version of a process for preparing an implant according to the invention, echogenic microcapsules are used as starting particles for the preparation of bubbles in the implant. The starting particles can be completely or partially retained as such after the preparation or after the implantation. It is however also conceivable that they change and are already no longer present on completion of the preparation or only some time after the implantation.

As the particularly echogenic microparticles (microcapsules) often have a certain sensitivity to strong pressures (e.g. greater than 0.5 bar) and sometimes also to increased temperature, it is important in these cases to select particularly gentle preparation processes for echogenic linear structures (e.g. filaments, threads) and pre-shaped bodies. For this, the following possibilities are listed as examples.

a) 2-phase encapsulation process using interfacial polymerization. Gas-filled microparticles are dispersed in an aqueous phase, the pH of which is set at a sufficiently basic value or which is buffered. In addition, one of the monomers (e.g. a diamine component) is dissolved in the aqueous phase, and the second monomer (e.g. a carboxylic acid dichloride) is dissolved in the lighter organic phase, which should be a non-dissolver for the microcapsules.

Because of their density, the echogenic microcapsules float in the direction of the phase interfaces. With a suitable pull-off, a thread in which the microcapsules are enclosed can be obtained.

This principle can also be transferred to other systems, such as e.g. other polyadditions, polycondensations or polymerizations. Equally suitable are other systems which can couple in aqueous systems to amines, thiols or alcohols and have at least two functional reactive groups from the groups: aldehydes, alcohols, semiacetals, anhydrides, acid halides, orthopyridyl disulfides, vinyl sulfones, epoxides, maleic acid imides, succimidyl esters, p-nitrophenyl carbonates, oxycarbonyimidazoles, benzotriazol carbonates, amines.

The location-stability of the microcapsules can be further increased by functional groups on the surface, for example, glass microcapsules can be surface-modified via reaction with 1,1,1-trialkoxysilyl amines or 1,1,1-trialkoxysilyl epoxides, a better and covalent incorporation into a filament matrix thereby being achievable. A similar procedure is also possible with surface-modified gas-filled polymermicrocapsules.

b) Solvent precipitation. A further possibility is to prepare an echogenic thread via a solvent precipitation and in so doing encapsulate the contrast agent. A suitable choice of solvent is important, especially in the case of sensitive polymer microcapsules. The solvent must not attack the capsule material.

A pH precipitation is advisable in particular for polyamides (e.g. nylon) or some proteins which are not soluble at neutral pH. This can be used e.g. in the case of gas-filled polybutylcyanoacrylate microcapsules such as described in WO 93/25242. Thus nylon can be dissolved in acid and the particles can be suspended in it and precipitated using a suitable precipitation bath.

c) Solvent evaporation. Echogenic pre-shaped bodies or threads can furthermore be prepared using a suspension of echogenic microparticles in a polymer solution. After the removal of the solvent via evaporation, the microparticles are enclosed. In this case also, the solvents should be selected so that damage to the particles by the solvent is very largely avoided for the time of thread and pre-shaped body manufacture.

d) Induced encapsulation. It is also possible to allow threads or pre-shaped bodies which are not soluble, but capable of expansion, under the prevailing conditions (e.g. solvent, pH, temperature) which are already either located on the basic structure of the implant or are subsequently applied to it to expand. The echogenic particles are applied to the implant, diffuse into it and are enclosed by returning the thread or pre-shaped body material to the initial state (e.g. removal of the swelling agent, pH change, temperature change).

e) Extrusion of filaments. As sometimes considerable pressures can occur in the case of single extruders or twin-screw extruders, those proposed by the manufacturer with sufficient pressure stability should be used in the case of glass hollow particles. The particle size to be used should be adjusted to the nozzle size.

f) Room-temperature encapsulation in hydrogel. Polymer microcapsules can be very gently encapsulated into hydrophilic polymer gels, as described below in Example 14, with low solvent content or solvent-free, such as e.g. prepared from hydroxyethyl methacrylate (HEMA), PEG acrylate, PEG methacrylates and their bifunctional derivatives. The polymerization preferably takes place under UV, optionally accelerated with sensitizing substances, such as dialkoxyphenyl acetophenones or in the presence of low-temperature initiators which allow a gentle processing both for the flexible basic structure of the implant and for the microcapsules.

To prepare resorbable pre-shaped bodies from hydrogel-containing resorbable echogenic microcapsules, monomers or prepolymers are preferably used such as are used in FocalSeal® (CAS no. 202935-43-1). In general, however, all hydrophilic resorbable bisacrylates or methacrylates are suitable for the preparation of hydrogels of the type: A-B-C-B-A with A = methacrylate, acrylate or vinyl ether, B = polylactide, polyglycolide, poly-2-hydroxybutyrate, poly-2-hydroxyvaleriate, polytrimethylene carbonate or their co-polymers, and C = a hydrophilic chain such as e.g. polyethylene glycol (PEG), polyvinyl alcohol (PVA) or polyvinyl pyrrolidone (PVP).

A further, particularly preferred possibility is to prepare echogenic polylactide microparticles in the presence of a protein via a spraying process. This is described below in Example 20 on the basis of Example 2 of DE 198 13 174 A1. Thus, for example, polyactide coglycolide particles (95/5)- prepared in the presence of albumin, in which the gas core is produced using the spraying process and not, as is customary, via a subsequent drying, can be resuspended in water after preparation and wetted by the addition of a dialdehyde such as e.g. glutaraldehyde. This can be carried out in a suitable mould which optionally also contains recesses and into which the basic structure of the implant, for example a mesh, is placed. As the pre-shaped bodies manufactured in this manner are themselves flexible and as a rule are anchored to the mesh over several stitches and the mesh is finally enclosed in the pre-shaped body, the implant with pre-shaped body has a sufficient stability such as is not achieved with many coating processes.

Depending on the intended use, it is advantageous if the implant according to the invention has at least one biologically active ingredient which can optionally be released locally after the implantation. Substances which can be considered for such an active ingredient are for example natural active ingredients, synthetic active ingredients, antibiotics, chemotherapeutics, cytostatics, metastasis inhibitors, antidiabetics, antimycotics, gynaecological agents, urological agents, antiallergics, sexual hormones, sexual hormone inhibitors, haemostyptics, hormones, peptide hormones, antidepressants, antihistamines, naked DNA, plasmid DNA, cationic DNA complexes, RNA, cell constituents, vaccines, cells occurring naturally in the body or genetically modified cells. The active ingredient can e.g. be present in encapsulated form or in adsorbed form, in particular on the basic structure or on ultrasonically detectable elements (e.g. pre-shaped bodies), special active-ingredient carriers also being conceivable. With such active ingredients, the diagnosis can be improved according to the application or a therapeutic effect can be achieved (e.g. better wound healing, inflammation inhibition).

In magnetic resonance tomography, areal polymer implants are normally visible. However, in particular in the case of light meshes which have a lower unit weight than polypropylene meshes customary in the trade, limitations can arise from the fact that very few protons of the implant material are present beside water and fatty protons of the body. To obtain a sufficient signal-to-noise ratio, in these cases, long measurement times, during which the patient must keep the respective body part still or, in the case of abdominal examinations, hold his breath, are necessary. In addition, if these implants are in the form of thin mesh strips, a typical scan depth of 6 mm can also already cause problems in recording the exact position and location of the implant.

In this case, the implants according to the invention have the advantage that, depending on the intended position in the body, fat-rich pre-shaped bodies can be attached to the implant for e.g. muscle implants or hydrous pre-shaped bodies for implants in a fatty environment. In addition, the hydrous pre-shaped bodies can also contain, as well as water, magnetic resonance contrast agents customary in the trade, such as e.g. "Endorem" (Guerbert), Gadolinium DTPA (Aldrich) or "Magnevist" (Schering).

Such pre-shaped bodies or also linear structures can be designed for example by applying polyethylene tubes filled with magnetic resonance contrast agent and having an internal diameter of 0.28 mm and an external diameter of 0.61 mm to a mesh. When measuring e.g. in a condensed-milk phantom (condensed milk plus gelatine) with a T2*-weighted gradient echo mode, both the contrast agent core and the polymer shell of the tube are clearly visible. In addition, the described ultrasonically detectable elements can be applied separately. It is also possible to react a suitable ultrasound contrast agent in aqueous phase with aqueous magnetic resonance contrast agent and to pour the mixture into a tube to thus form a pre-shaped body. Alternatively, these contrast agents can be applied to the implant in a sufficiently cross-linked gel from which the contrast agent cannot diffuse out. Furthermore, the encapsulated fluoroalkanes detectable in the ultrasound are also suitable to achieve a magnetic resonance contrast.

For implants according to the invention constructed in this way, a particularly specialized magnetic resonance system such as described in Paley et al. (Eur. Radiol. 7, 1431-1432 (1997)) is not necessary. Equipment customary in the trade is sufficient and the radiologist achieves good results with settings as already pre-set in the equipment for example for meniscus examinations. A special coating as described by Paley et al. (superparamagnetic ferric oxide enclosed in a polystyrene film) is likewise not necessary with the pre-shaped bodies or linear structures mentioned above.

It is also conceivable to provide on an areal implant exclusively elements which are set up for detectability in magnetic resonance and do not improve the visibility of the implant in the ultrasound. Such elements can e.g. be constructed as a tube filled with magnetic resonance contrast agent, as described above.

In the following, the invention is explained using embodiments. Further possibilities for the implant according to the invention and processes for preparing it emerge directly from the claims. The drawings show in
- Figure 1: a schematic top view of the implant prepared according to example 2,
- Figure 2: an ultrasound view of the implant according to example 2 after implantation in a pig's stomach,
- Figure 3: an ultrasound view of a marked filament according to example 3,
- Figure 4: a cross-section through a pre-shaped body of the implant prepared according to example 7,
- Figure 5: a section from the filament prepared according to example 8, seen in side view,
- Figure 6: a schematic representation of the pattern draft of the fabric prepared according to example 9,
- Figure 7: a schematic top view of the implant prepared according to example 10 and
- Figure 8: a schematic top view of the implant prepared according to example 15.

In the following, trade names are generally indicated by quotation marks, as before. A respective trade name might involve registered trade marks.

### Example 1: Circular pre-shaped bodies made from integral foam on polypropylene meshes

3 foam pieces (3M Foam Medical Tapes no. 1773, 30 mil; closed-cell polyethylene foam 0.87 mm thick) were attached at a distance of 3.5 cm from each other in the middle to a polypropylene mesh customary in the trade measuring 1.1 cm * 45 cm, as used in a so-called TVT system of the manufacturer Medscand Medical AB. The round foam pieces were punched out beforehand (diameter 0.5 cm). Attachment was by ultrasound welding from the mesh side.

A cut approximately 2 cm deep was made over the whole width, approximately 4 cm from the edge, in a piece of pig's stomach. The mesh strip was coated with contact gel and inserted. Sounding was carried out from the side with a Toshiba ultrasound apparatus with a sound head of 3.75 MHz. While the mesh was scarcely or only very weakly recognizable, the pre-shaped bodies were clearly recognizable and above all clearly distinguishable from other structures.

A mesh piece which was kept beforehand for 3 months in phosphate buffer of pH=7.0 at 38°C also showed a comparable contrast.

### Example 2: Annular oval pre-shaped body made from integral foam on polypropylene mesh

A foam piece (3M Foam Medical Tapes no. 1773, 40 mil; closed-cell polyethylene foam 1.02 mm thick) was attached in the middle to a polypropylene mesh customary in the trade measuring 1.1 cm * 45 cm, as used in a so-called TVT system of the manufacturer Medscand Medical AB. The foam piece was cut out as an oval beforehand (length 1.3 cm, width 0.8 cm) and provided with a central perforation (diameter 0.5 cm). Attachment was carried out by ultrasound welding from the mesh side.

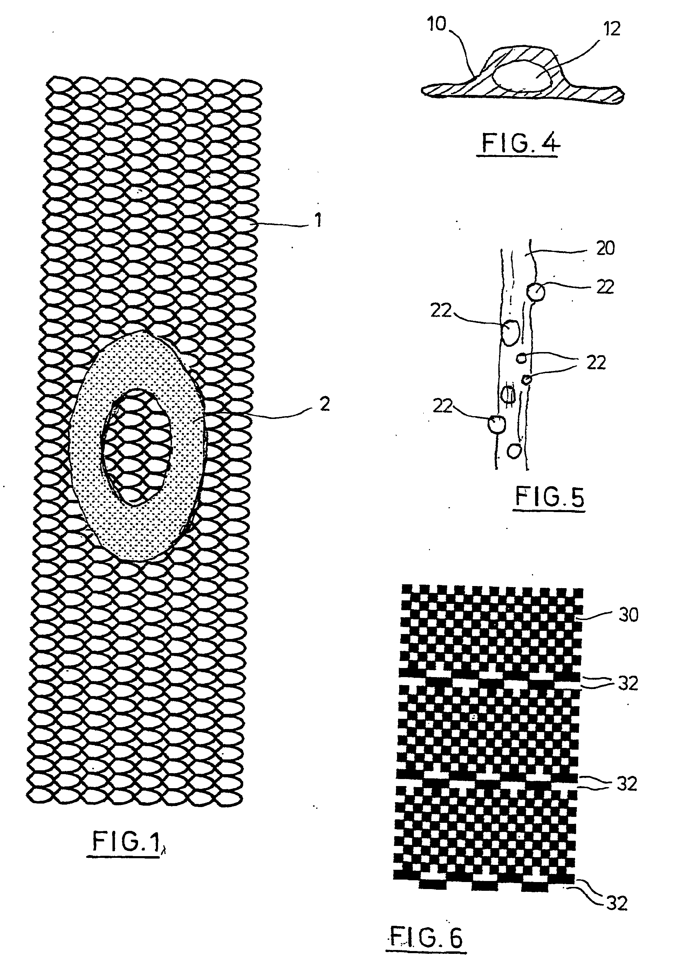

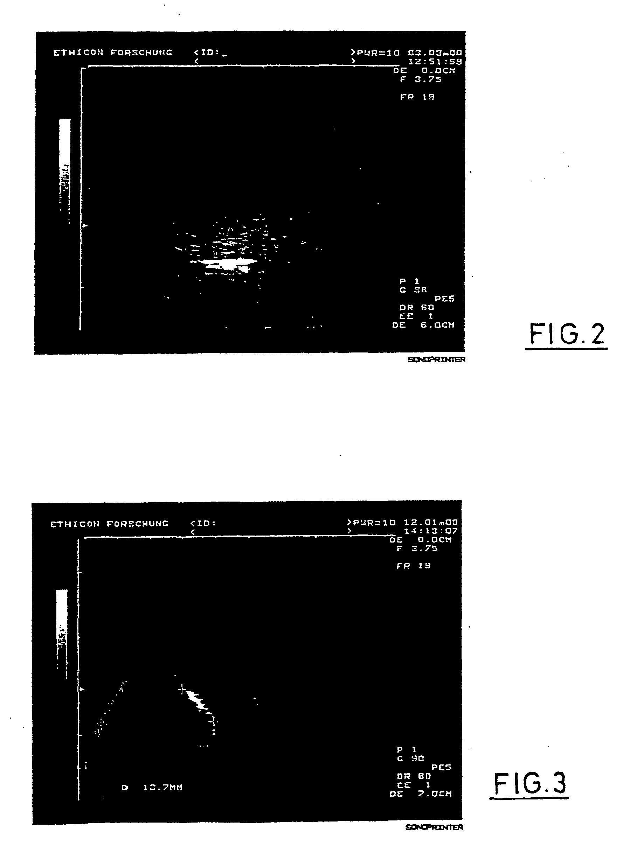

Figure 1 shows a schematic top view of the implant. In it, the polypropylene mesh which serves as a flexible basic structure is numbered 1 and the echogenic pre-shaped body made from polyethylene foam 2.

A cut approximately 2 cm deep was made over the overall width, approximately 4 cm from the edge in a piece of pig's stomach. The mesh strip was coated with contact gel and inserted. Sounding was carried out from the side with a Toshiba ultrasound apparatus with a sound head of 3.75 MHz. While the mesh was scarcely or only very weakly recognizable, the foam was clearly visible and above all clearly distinguishable from other structures. Figure 2 shows an ultrasound view of the implant inserted into the pig's stomach.

### Example 3: Sealed hollow threads on polypropylene threads

Hollow polyimide microfibres were wound onto a 0.3 mm thick polypropylene filament at 5 cm intervals to a width of approximately 1.3 cm (internal diameter 0.1 mm, wall thickness 13 *µ*m, manufacturer MicroLumen) so that a double winding resulted. These regions were fixed with "Histoacryl" (B. Braun Surgical GmbH) and then sealed with paraffin wax (melting point 73-80°C) . These marked filaments can also be incorporated into meshes as stationary threads in the crochet galloon technique.

Whereas the polypropylene filament was hardly visible in the ultrasound, the markings were clearly recognizable. Figure 3 shows an ultrasound view of the marked filament.

### Example 4: Glass hollow bodies on polypropylene mesh

A mixture of approximately equal volumes of glass hollow bodies (Scotchlite® K1, 3M) and paraffin wax was manufactured and homogenized by melting and stirring. The warm mixture was poured into a cool glass mould. The solidified film (syntactic foam) had a height of approximately 1 mm. Strips approximately 2 mm wide and 0.8 cm long were cut with a scalpel. These were laid onto a 45 cm long and 1.1 cm wide polypropylene mesh. Small pieces were taken from these strips, shaped to a small ball and pressed onto the mesh. The marking had a length of approximately 2 mm, a width of approximately 1 mm and a height of approximately 0.7 mm. The marking was then reacted with some drops of a 2% polycarbonate solution ("Makrolon", Bayer AG) in chloroform. After the removal of the solvent by evaporation, the marking was incorporated in the polymer film and could not be removed from the mesh by vigorous mechanical rubbing. In this way, markings were applied at a distance of 1.5 cm from centre to centre.

The markings showed a clear contrast in the B image and red- and blue-coded pixels in the colour-doubler image (UM9 ultrasound equipment from ATL).

### Example 5: Encapsulated hollow threads on polypropylene mesh

The procedure was as in example 4 with the difference that, instead of hollow glass balls, cut hollow threads were used (Hollofil® , type no. 4H, DuPont).

These markings showed a clear contrast in the B image, but no colour doubler effects whatsoever.

### Example 6: Welded hollow polyethylene pre-shaped bodies on composite mesh

Polyethylene tube pieces closed at the ends were welded with ultrasound, 5 cm from each other, to a non-resorbable, experimental woven product made from polypropylene (Prolene®, Ethicon) and a mixture of polyvinylidene fluoride and polyhexafluoropropylene (Pronova®, Ethicon) . The woven product (mesh) was prepared on a Raschelina RD3MT3/420SN type crochet galloon machine. The mesh is a large-pored open mesh made from polypropylene yarns with additional coloured broché threads made from a "Pronova" monofilament of 0.15 mm diameter. The welding to the mesh was carried out from the mesh side at the flattened tube ends.

The sealed tube pieces were manufactured as follows: A polyethylene tube piece approximately 3 cm long (ref. 800/1000/420/100, Sims Portex) was held for several seconds on both sides at 120°C in a compression press without additional pressure. The flatted and melted ends were cut to size to a length of approximately 3 mm each. The gas-filled core piece had a length of 7 mm and a core diameter of 1.57 mm.

### Example 7: Heat-sealed hollow polyethylene pre-shaped bodies on composite mesh

Polyethylene tube pieces closed at the ends were welded with ultrasound 2 cm from each other, to a non-resorbable, experimental woven product made from polypropylene and Pronova (see example 6). The mesh was prepared on a Raschelina RD3MT3/420SN type crochet galloon machine. The mesh is large-pored open mesh made from polypropylene yarns with additional coloured broché threads made from Pronova # 5-0 monofilament. The welding to the mesh was carried out from the mesh side at the flattened tube ends.

The sealed tube pieces were manufactured as follows: A polyethylene tube piece approximately 3 cm long (ref. 800/1000/420/100, Sims Portex) was kept for several seconds on both sides at 120°C in a compression press without additional pressure. The flattened and melted ends were cut to size to a length of approximately 2 mm each. The gas-filled core piece had a length of 3 mm and a core diameter of 0.28 mm.

Figure 4 shows a cross-section through the echogenic pre-shaped body which is formed by a tube piece 10 closed at both ends. The cutting plane lies in the region of the gas-filled core 12.

### Example 8: Echogenic propylene filaments with pressure-sensitive glass hollow bodies

A mixture of polypropyelene granules containing 1 wt. -% glass hollow bodies (Scotchlite® K1, 3M) was prepared. This mixture was melted and mixed vigorously with a glass rod. A thread approximately 1 m long was pulled out with the glass rod. This had a microscopic thickness of 0.15 mm. Under the microscope, the intact glass hollow bodies (glass microcapsules) were very clearly recognizable in the filament.

Figure 5 shows a section from the filament 20 in side view. A section of the glass hollow body 22 is only partially surrounded by polypropylene and projects, the remaining section is on the other hand completely encapsulated.

In the water bath, the filament showed a clearly greater contrast in the ultrasound than a thread of comparable thickness made from polypropylene.

### Example 9: Fabric with echogenic film strips

A part of the cured composite from example 8 consisting of 1% Scotchlite K1 (3M) and polypropylene (Ethicon Inc.) was kept in a heating press for a period of 30 minutes between backing paper at 180°C. The resultant film was then subjected for 2 minutes to an external pressure of 3 bar and kept once more for 15 minutes at 180°C without external pressure. The composite film subsequently had a thickness of 0.58 mm. Strips with a width of 3 mm were punched out with a punching mould.

The film strips were woven out as weft threads in a dobby loom as effect threads in a combined weave. Polypropylene yarns of 60 den were used for the warp and weft threads in the backing fabric. A plain weave was selected for the backing fabric and the echogenic film strip described above was inserted twice as a rep-weave weft thread after every tenth weft insertion in the plain weave.

Figure 6 shows the structure of the fabric in schematic form, the backing fabric (flexible basic structure) being numbered 30 and an echogenic film strip 32.

### Example 10: Implant mesh with echogenic filaments which contain gas-filled microcapsules

A mixture of 2.5 wt.-% glass hollow bodies (Scotchlite ® SK 38, 3M) and the polymer polypropylene (basic material for Prolene ®, Ethicon Inc.) was extruded at 230°C in a Haake extruder with melt pump and multi-bore nozzle. Filaments 0.2 mm thick were obtained.

These echogenic threads were processed on a Raschelina RD3MT3/420SN type crochet galloon machine together with polypropylene yarns. The polypropylene yarns served as core threads and the echogenic threads were worked in as broché threads during the manufacturing process.

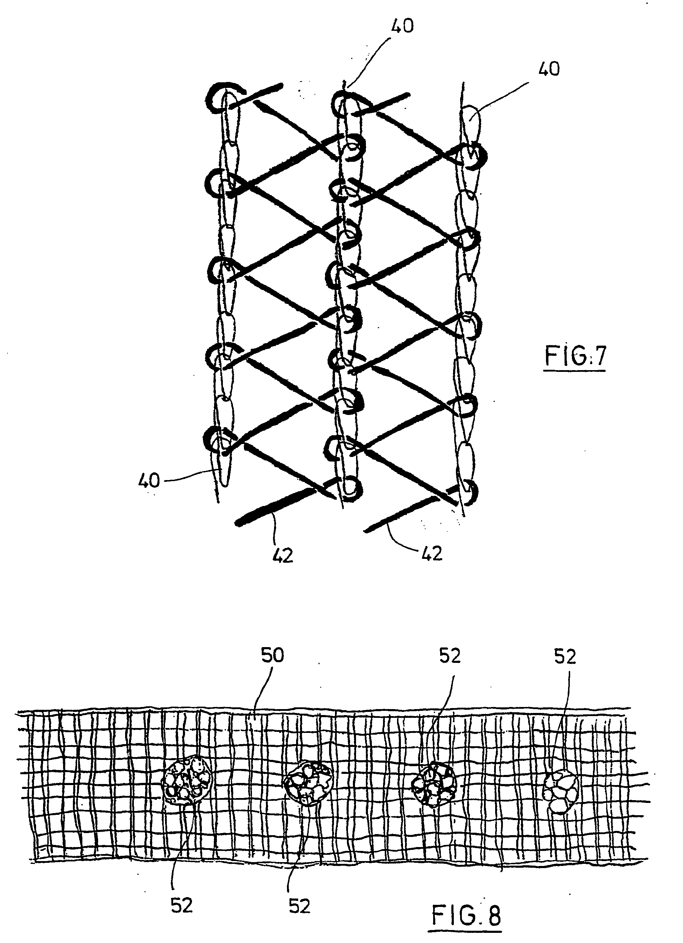

Figure 7 shows a section from the woven product with the core threads 40 made from polypropylene and the echogenic broché threads 42. The ultrasonically detectable elements, namely the broché threads 42, are thus worked into the flexible basic structure of the implant as a structural component in this example, forming the implant mesh together with the core threads 40.

### Example 11: Echogenic pre-shaped bodies made from polyethylene

Pre-shaped bodies with a core length of 1.5 mm and a core diameter of approximately 0.58 mm were manufactured from a polyethylene tube (ref. 800/110/100, Sims Portex, internal diameter 0.28 mm, external diameter 0.61 mm) with the help of a brass stencil.

To this end, the stencil was first manufactured by pressing zirconium dioxide balls with 1.5 mm diameter (Mühlmeier Mahltechnik) between two brass sheets approximately 0.75 cm apart in a compression press at approximately 5 bar pressure. After the balls were removed, their impressions were found in a line on the two sheets with a maximum diameter of 1.5 mm and a depth of approximately 0.75 mm.

A tube piece (ref. 800/110/100, Sims Portex, internal diameter 0.28 mm, external diameter 0.61 mm) was laid onto one of the sheets and fixed to the right of the impressions with some Sellotape (Beiersdorf AG). The compression press was heated to 120°C, then the sheet with affixed tube piece and some backing paper kept under pressure of 1 bar for a few seconds. After removal, a film of approximately 0.28 mm thickness and approximately 1.5 mm width was removed which had gas-filled elements with a length of approximately 1.5 mm and height and width of approximately 0.6 mm in intervals of 0.75 cm.

These pre-shaped bodies were pressed vigorously with the fingers in a water bath without gas escaping or water entering. The gas-filled pre-shaped bodies were cut to fit the film pieces and sewn in parallel-arranged lockstitches using star-shaped topstitching.

### Example 12: Pre-shaped bodies made from polyethylene tube with gas-filled microcapsules made from glass on polypropylene mesh

A cold mixture of 20 g surfactant ("Pluronic F127", "Lutrol F127", BASF) with 2.5 g glass hollow bodies (Scotchlite® K1, 3M) in 75 g water was prepared. This was enclosed in Portex polyethylene tubes using knots 1.5 cm apart. The projecting ends beside the knots were thermally sealed at approximately 120°C to a polypropylene mesh serving as a flexible basic structure. The distance between the pre-shaped bodies (centre to centre) was 2.5 cm.

### Example 13: Stabilized gas-filled microcapsules on polypropylene mesh

Echogenic, decomposable microparticles were prepared according to example 9 of EP 0 644 777 B1 without diluting these in sodium chloride and Cetomakrogol. The microparticles were diluted 1 to 10 after preparation in cold, acidified surfactant solution (" Pluronic F127" , BASF; 20%) and poured into a polyethylene tube (Sims Portex, 0.28 mm internal diameter, 0.61 mm external diameter, ref. 800/110/100) into which some Panacryl® threads (Ethicon GmbH) of a length of 0.5 cm had already been drawn beforehand. Panacryl® is a resorbable suture material and decomposes slowly into the components lactic acid and glycolic acid. The tube ends were then knotted at intervals of approximately 1 cm and the tube ends thermally sealed on the other side of the knots to a mesh made from polypropylene.

The mesh was kept in a phosphate buffer at pH=7 for 6 months at 38°C in the thermostat. Even after this time, the echogenic marking in the form of the filled tube were still clearly recognizable in the B image and the colour doubler (red and blue coding) of an ultrasound apparatus.

### Example 14: Polypropylene tape, enclosed in sections in hydrogel, with long-term stable polymer microcapsules

In this example, there is described the preparation of long-term-stable, echogenic microcapsules and their gentle encapsulation at room temperature in a biocompatible, long-term-stable hydrogel, which is firmly anchored to an implant tape.

Echogenic microparticles were prepared as in example 13, only the monomer was exchanged for octylcyanoacrylate (Dermabond®, Ethicon) and the pH value was kept at neutral (no pH setting) with a reaction time of 2 hours. Even after approximately 4 months' storage at room temperature, the suspension still showed a comparatively high level of floating material, as at the beginning of the storage.

A monomer/solvent mixture was prepared by adding 20 ml hydroxyethyl methacrylate (HEMA, Opthalmic Grade, Polysciences LTD), 110 mg 2,2 dimethoxy-2-phenylacetophenone (Aldrich, 24650-42-8), 10 ml isopropanol and 0.5 ml ethylene glycol dimethacrylate (Polysciences LTD) to 60 ml polyethylene glycol 300. After a period of time, a clear solution formed.

A mould was made from beeswax in an aluminium bowl. To do this, beeswax was melted in the aluminium bowl. Three metal rods with a diameter of 5 mm were inserted into the mould. After cooling, the rods were removed. Recesses of approximately 5 mm depth resulted. A polypropylene tape (mesh) as used for the commercial product "TVT" was then laid over the three recesses and fixed in the wax with 2 needles.

Then 50 ml of the prepared monomer solution was reacted with 1 ml floating material of the polyoctylcyanoacrylate suspension stored in neutral for 4 months and briefly dispersed with a magnetic stirrer. The monomer/particle mixture was poured into the mould and irradiated for half an hour with a UV polymerization lamp (Polysciences, catalogue no. 24001) at a distance of approx. 15 cm.

After careful removal of the tape from the mould, it was seen that the tape was enclosed on both sides in a gel pre-shaped body which consisted of a foot measuring approx. 5 cm* 1.5 cm * 3 mm. The tape lay approximately in the middle of the 3 mm thick gel sheet. Cylindrical caps approximately 5 mm high and approximately 5 mm in diameter were situated at a distance of approximately 1.5 cm from each other, dictated by the mould. Intact microparticles were recognizable in the gel body under a light microscope.

The tape with the gel marking was then washed for several days with distilled water, the water being changed daily.

### Example 15: "Panacryl" film with gaseous inclusions on " Panacryl" tape

Gas-containing pre-shaped bodies in the form of films were applied to a slowly resorbable "Panacryl" tape (Ethicon), manufactured on a Tascheline RD3MT3/420SN type crochet galloon machine from 80 den multifilament threads with a width of just 2 cm. "Panacryl" (Ethicon) is a polylactide coglycolide in the ratio 95/5.

For this, the tape was laid onto a PTFE-coated sheet. In the middle of the tape, at distance of approximately 1.5 cm from each other, drops of a 5% solution of 95/5 polylactide coglycolide as also used in Panacryl® (Ethicon GmbH) are added in each case to chloroform. The sheet was heated for several minutes to 70°C. Round film pieces formed with numerous bubble-shaped inclusions in the tape with approx. 5 mm diameter. The filament pieces of the tape were enclosed by the film on both sides. In the centre of the film, the tape had dissolved. Despite this, the gas-containing film pieces were so firmly anchored to the tape that they could not be removed mechanically by rubbing.

Figure 8 shows a top view of a section of the tape 50 with the gas-containing film pieces 52.

### Example 16: Preparation of gas-filled films made from polycarbonates as pre-shaped bodies

A 10% solution of polycarbonate ("Makrolon", Bayer AG) in chloroform was prepared. On a brass sheet (approx. 1 cm thick), the 1-mm-high polymer solution was applied with a slider with a 1-mm-deep indentation. The thus-coated sheet was laid for several minutes on a heating plate (100°C) and cold air was passed over it from time to time. A polymer film formed with numerous gas inclusions of approximately 0.1 to 3 mm diameter. The bubbles lay close together and in one layer.

Round objects with a diameter of 4 mm were punched out and these were heat-sealed with polypropylene meshes in the ultrasound.

### Example 17: Preparation of film-like pre-shaped bodies from echogenic microparticles and silicone

A silicone educt mixture of 10 parts component A ("Essil 244 A2", Axson) and 1 part component B ("Essil 244 B" , Axson) was applied thinly with a brush transversely onto a polypropylene mesh strip with a width of approximately 1 cm. Strips were produced with approximately 1 cm width and at a distance of approximately 2.5 cm from each other, which filled the meshes.

In a second step, some substance was taken with a thin glass rod from the floating material of the echogenic microparticle mixture from example 13, which had formed after approximately 1 week and had a solid, creamy consistency, and spread onto the individual silicone strips. Some more silicone starter mixture was then added to these strips with the obtained microparticle markings of approximately 0.5 cm diameter. After overnight curing, flexible rubber-like film strips had formed which contained, in the centre, bubbles measuring approximately 0.05 mm to 1 mm. In addition, microscopic inclusions with a diameter of approx. 50 *µ*m were observed which contained microparticles measuring 1 to 2 *µ*m.

### Example 18: Preparation of film-like pre-shaped bodies with echogenic microparticles

The microparticle suspension prepared in example 13 was resuspended by vigorous shaking and diluted 1 to 20 in water. The silicone starter mixture from example 17 was then painted onto a PTFE-coated metal sheet, reacted with 1 ml of the diluted microcapsule suspension, this was painted over its whole surface (approx. 8 cm * 8 cm) and coated again with starter mixture. The resulting film was kept overnight at room temperature. Bubbles 0.05 to 1 mm in size formed, distributed evenly over the film.

### Example 19: Preparation of slowly resorbable pre-shaped bodies with resorbable echogenic microparticles

Butylcyanoacrylate (Sichel GmbH) was added dropwise into an aluminium bowl with a flat base so that a liquid film of approximately 3 cm * 3 cm formed. Approximately 6 drops of the undiluted acid suspension from example 13 was then added and the mixture was left to stand overnight. The next morning, a homogenously cloudy film had formed with a clear border of approximately 1 to 2 mm. At the points where the microcapsule drops had been located, areas of thicker, cloudier film were observed. The film had a thickness of approximately 0.75 mm and in the region of the thicker areas a thickness of approximately 2 mm. Under the microscope, microcapsules were to be recognized in the whole film. In contrast to examples 17 and 18, almost no macroscopically visible bubbles had formed.

### Example 20: Preparation of slowly resorbable pre-shaped bodies with resorbable echogenic microparticles and attachment to a partially resorbable composite mesh

Gas-filled microcapsules were prepared on the basis of De 198 13 174 Al, example 2, but from a copolymer made from 95 parts polylactide and 5 parts glycolide (Panacryl®, Ethicon Inc).

A mould was prepared from the same polymer by adding polymer granules to a brass sheet which every second millimetre contained square raised areas measuring 1 mm * 1 mm and 0.5 mm height. A level sheet was laid on and the polymer granules were melted on, exerting light manual pressure, above 200°C. The mould was quenched under water and the film removed. The film had a thickness of 1 mm with equidistant recesses of 0.5 mm. The powder obtained in the first step from the microcapsules was introduced into the recesses with a brush. A second film made from the same polymer with a thickness of 50 *µ*m was expanded in chloroform and glued to the first film under slight pressure. A perforator was used to punch out pre-shaped bodies in the shape of round film pieces with a diameter of approximately 6 mm.

The film pieces were laid out 3 cm apart in a PTFE-coated trough and covered with an implant mesh customary in the trade ("Vypro", Ethicon GmbH) made from polypropylene yarn and a copolymer of glycolide and lactide in the ratio 90 to 10 (Vicryl®, Ethicon) . A 10% (wt.-%) solution of polycarbonate (" Makrolon" , Bayer AG) in chloroform was then dropped onto the film pieces, so that the film pieces did not dissolve and were connected to the mesh via the polycarbonate film.

### Example 21: Preparation of a slowly resorbable film with gas inclusions and connection of pre-shaped bodies manufactured therefrom to a partially resorbable mesh

A 5% (wt.-%) solution in chloroform was prepared from a polylactide coglycolide 95/5 (Panacryl®, Ethicon Inc.). 50 ml of this solution was shaken vigorously by hand for several minutes and then stirred with an IKA "Ultraturrax" stirrer at 5000 revolutions per minute. The bubble-containing, viscous solution was poured into a PTFE-coated mould (fill level approx. 1 mm) and kept for 1.5 hours at approximately 50°C heating-plate temperature. A very flexible film approximately 0.25 mm thick formed in which bubbles were enclosed, evenly distributed in for the most part a single layer (the majority with a diameter of 0.5 to 1 mm). However, bubbles smaller than 0.1 mm and also some with a diameter of 5 mm were also observed under the microscope.

Round pieces with a diameter of 5 mm were punched out. These were laid 2.5 cm apart onto a sheet coated with PTFE. A 4 cm * 11.5 cm composite mesh comprising a 90/10 polyglycolide colactide and polypropylene ("Vypro", Ethicon GmbH) was laid onto these film pieces. The mesh was coated with the 5% polylactide coglycolide solution with a paintbrush in the region of the film pieces and a second punched-out film piece was placed onto each as a counterpiece. The film pieces were briefly pressed together manually. The film pieces did not dissolve, rather they merely stuck together.

The gas-filled pre-shaped bodies manufactured in this way had a diameter of approx. 6 mm and a thickness of approx. 0.5 mm and were so firmly anchored to the mesh that they could not be removed from the mesh by manual bending, pulling apart or rubbing.

### Example 22: Preparation of a slowly resorbable film with resorbable echogenic microcapsules on a slowly resorbable tape

The procedure was as in example 15, except that the formation of the film involved a different emulsion. For this, an emulsion consisting of approx. 2 ml SPAN80® (sorbitan monooleates, Sigma), 5 ml 5% solution of polylactide-coglycolide which is also used in Panacryl® (Ethicon GmbH) in chloroform and approximately 0.5 ml suspension from example 13 was prepared by simple manual shaking. The film formation took place analogously to example 15 on the tape at 40°C.

### Example 23: Pre-shaped bodies made from syntactic foam on polypropylene tape

Disks with a diameter of 0.5 cm were punched out from the composite film prepared in example 9. These were heat-sealed 1.5 cm apart in a row from the mesh side with ultrasound onto a mesh-like polypropylene tape customary in the trade ("TVT"-tape", Medscand Medical AB).

The disks could not be removed from the tape by mechanical means. Nor could a perceptible change in elasticity or flexural strength be ascertained between the region marked with the disks and the unmarked region.

### Example 24: Pre-shaped bodies which additionally contain magnetic resonance contrast agents on polypropylene tape

A polyethylene pre-shaped body partially filled with magnetic resonance contrast agent was prepared. For this, an approximately 3-cm long polyethylene tube piece (ref. 800/110/100, Sims Portex, internal diameter 0.28 mm, external diameter 0.61 mm) was kept for several seconds on one side at 120°C in a compression press without additional pressure. Some magnetic resonance contrast agent (Endorem®, Guerbert) was then poured in to a height of approx. 5 mm. The second tube side was then thermally sealed. The flattened and melted ends were cut to size to a length of approximately 5 mm each. The core piece filled with gas and magnetic resonance contrast agent had a length of approximately 1 cm.

The pre-shaped body was sealed with ultrasound from the mesh side onto a polypropylene mesh piece.

The mesh marked with the pre-shaped body was enclosed in a condensed-milk/gelatine phantom (6 g gelatine in 200 ml 7% condensed milk) and measured with a "Vista MRT" magnetic resonance apparatus (1 tesla). The tube piece was clearly recognizable in a T2*-weighted gradient echo sequence, as used for meniscus examinations.

## Claims

1. Areal implant, with a flexible basic structure on a polymer basis (1; 30, 32; 40, 42; 50) and with ultrasonically detectable elements (2; 10; 20; 32; 42; 52), which contain or produce gas and which are set up for detectability for at least four weeks after implantation.

2. Implant according to claim 1, **characterized in that** the elements (52) detectable in ultrasound are arranged in an areal pattern.

3. Implant according to claim 1 or 2, **characterized in that** the basic structure (1; 30; 40) contains non-resorbable polymer.

4. Implant according to claim 3, **characterized in that** the basic structure (1; 30; 40) contains at least one of the substances selected from the following group: polyalkenes, polypropylene, polyethylene, partially halogenated polyolefins, wholly halogenated polyolefins, fluorinated polyolefins, polytetrafluorethylene, polyvinylidene fluoride, polyisoprenes, polystyrenes, polysilicones, polycarbonates, polyarylether ketones, polymethacrylic acid esters, polyacrylic acid esters, polyimides, copolymers of polymerizable substances thereof.

5. Implant according to one of claims 1 to 4, **characterized in that** the basic structure (50) contains resorbable polymer.

6. Implant according to claim 5, **characterized in that** the basic structure (50) contains at least one of the substances selected from the following group: polyhydroxy acids, polylactides, polyglycolides, polyhydroxy butyrates, polyhydroxy valeriates, polycaprolactones, polydioxanones, synthetic and natural oligo- and polyamino acids, polyphosphazenes, polyanhydrides, polyorthoesters, polyphosphates, polyphosphonates, polyalcohols, polysaccharides, polyethers, polyamides, aliphatic polyesters, aromatic polyesters, copolymers of polymerizable substances thereof, resorbable glasses.

7. Implant according to one of claims 1 to 6, **characterized in that** the basic structure has one of the forms selected from the following group: meshes (1), tapes (50), films, perforated films, felts, fleeces, open-pored foam films.

8. Implant according to one of claims 1 to 7, **characterized in that** the elements (2) detectable in ultrasound contain at least one of the substances selected from the following group: physiologically acceptable gases; low-boiling-point liquids which are present in gaseous form at 38°C; low-boiling-point liquids which vaporize in the ultrasound field.

9. Implant according to claim 8, **characterized in that** the elements (2; 10; 20; 32; 42; 52) detectable in ultrasound contain at least one of the substances selected from the following group: non-, partially and perfluorinated n-, iso-, neo- and cycloalkanes, fluorobromoalkanes, sulfur hexafluoride, hydrogen, nitrogen, oxygen, air, carbon dioxide, helium, neon, argon, xenon, krypton.

10. Implant according to one of claims 1 to 9, **characterized in that** the elements (2; 10; 20; 32; 42) detectable in ultrasound contain a non-resorbable structural material.

11. Implant according to claim 10, **characterized in that** the structural material of the elements (2; 10; 20; 32; 42) detectable in ultrasound contains at least one of the substances selected from the following group: polyalkenes, polypropylene, polyethylene, partially halogenated polyolefins, wholly halogenated polyolefins, fluorinated polyolefins, polytetrafluorethylene, polyvinylidene fluoride, polyisoprenes, polystyrenes, polysilicones, polycarbonates, polyarylether ketones, polymethacrylic acid esters, polyacrylic acid esters, polyimides, hydrophilic cross-linked polymers, silicones, copolymers of polymerizable substances thereof, ceramics, glasses, metals, carbon.

12. Implant according to one of claims 1 to 11, **characterized in that** the elements (52) detectable in ultrasound contain a resorbable structural material.

13. Implant according to claim 12, **characterized in that** the structural material of the elements (52) detectable in ultrasound contains at least one of the substances selected from the following group: polyhydroxy acids, polylactides, polyglycolides, polyhydroxybutyrates, polyhydroxyvaleriates, polycaprolactones, polydioxanones, synthetic and natural oligo- and polyamino acids, polyphosphazenes, polyanhydrides, polyorthoesters, polyphosphates, polyphosphonates, polyalcohols, polysaccharides, polyethers, polyamides, aliphatic polyesters, aromatic polyesters, natural polyamino acids, synthetic polyamino acids, genetically produced polyamino acids, collagen, rhcollagen, silk, pseudopolyamino acids, polycyanoacrylates, polyethylene glycols, polyvinyl alcohols, derivatized cellulose, fats, waxes, fatty acids, fatty acid esters, polyphosphate esters, copolymers of polymerizable substances thereof, resorbable glasses.

14. Implant according to one of claims 1 to 13, **characterized in that** at least part of the ultrasonically detectable elements is formed as pre-shaped bodies (2; 10; 52) with respective length, width and height in the range from 0.1 mm to 50 mm.

15. Implant according to claim 14, **characterized in that** at least one pre-shaped body has one of the shapes selected from the following group: rings (2), disks, platelets, buttons, flat ellipses, hemispheres, solid spheres, beads, cylinders, cubes, blocks, cones, rods, sleeves, tubes (10), pipes, films (52).

16. Implant according to one of claims 1 to 15, **characterized in that** at least part of the ultrasonically detectable elements is designed as linear structures (20; 32; 42).

17. Implant according to claim 16, **characterized in that** at least one linear structure is of a type selected from the following group: tapes, cords, threads (20; 40), twines, knotted filaments, film tapes (32), covering twines.

18. Implant according to one of claims 1 to 17, **characterized in that** at least part of the ultrasonically detectable elements is connected to the basic structure in at least one of the ways selected from the following group: melting on, welding (2; 10), application from solution (52), gluing, knotting, attachment to a holding device connected to the basic structure, incorporation into the basic structure using textile techniques (32; 42).

19. Implant according to one of claims 1 to 18, **characterized in that** at least part of the ultrasonically detectable elements has a symbol detectable in ultrasound, which is preferably provided repeatedly at uniform intervals.

20. Implant according to claim 19, **characterized in that** the symbol is designed in one of the forms selected from the following group: sewn from linear structures, embroidered from linear structures, embossed from film, composed of several objects.

21. Implant according to one of claims 1 to 20, **characterized in that** at least part of the elements (2) detectable in ultrasound has a structure with a mono-to-multi-cell integral foam, the internal walls of which are dry or wetted by a liquid.

22. Implant according to one of claims 1 to 21, **characterized in that** at least part of the elements (20; 32; 42) detectable in ultrasound contains a matrix into which gas-filled microcapsules (22) are embedded.

23. Implant according to claim 22, **characterized in that** the microcapsules are present in at least one of the ways selected from the following group: microcapsules surrounded by liquid which are embedded in polymer; microcapsules surrounded by liquid which are embedded in fat; microcapsules surrounded by liquid which are embedded in an organo-gel; microcapsules surrounded by liquid which are embedded in a glass; microcapsules (22) which are embedded in a hydrophobic polymer; microcapsules which are embedded in a hydrophilic gel; microcapsules which are embedded in a cross-linked polymer; microcapsules which are embedded in a polymer gel; microcapsules which are embedded in an open-pored polymer foam, the diameter of the microcapsules generally being greater than the pore diameter of the outer foam pores.

24. Implant according to one of claims 1 to 23, **characterized in that** at least part of the ultrasonically detectable elements has a structure with a composite which contains hollow threads in a polymer matrix.

25. Implant according to one of claims 1 to 24, **characterized in that** at least part of the ultrasonically detectable elements has a structure with open-pored pre-shaped bodies or linear structures which are surrounded by a gas-tight envelope.

26. Implant according to one of claims 1 to 25, **characterized in that** at least part of the ultrasonically detectable elements is composed at least partially of gas-filled microparticles, the microparticles being surface-fused, i.e. preferably thermally surface-filmed, ionically crosslinked and/or chemically crosslinked.

27. Implant according to one of claims 1 to 26, **characterized in that** at least part of the elements (52) detectable in ultrasound comprises a structure with blister films.

28. Implant according to one of claims 1 to 27, **characterized in that** the implant is also detectable in magnetic resonance tomography.

29. Implant according to claim 28, **characterized by** pre-shaped bodies or linear structures which favour a magnetic resonance contrast, the pre-shaped bodies or linear structures preferably comprising tubes filled with water, magnetic resonance contrast agents and/or fat.

30. Implant according to one of claims 1 to 29, **characterized by** at least one biologically active ingredient which preferably comprises at least one of the substances selected from the following group: natural ingredients, synthetic ingredients, antibiotics, chemotherapeutics, cytostatics, metastasis inhibitors, antidiabetics, antimycotics, gynaecological agents, urological agents, anti-allergic agents, sexual hormones, sexual hormone inhibitors, haemostyptics, hormones, peptide hormones, antidepressants, anti-histamines, naked DNA, plasmid DNA, cationic DNA complexes, RNA, cell constituents, vaccines, cells occurring naturally in the body, genetically modified cells.

31. Implant according to claim 30, **characterized in that** the active ingredient is present in at least one of the forms selected from the following group: in encapsulated form, in adsorbed form, in the basic structure, at the basic structure, in ultrasonically detectable elements, at ultrasonically detectable elements.

32. Process for producing an implant according to claim 1, **characterized in that**, during preparation of the ultrasonically detectable elements, structural material of the ultrasonically detectable elements is extruded and, in doing so, a gas is introduced into the structural material by direct gassing or under supercritical conditions, the gas preferably containing at least one of the substances selected from the following group: air, carbon dioxide, nitrogen, mixtures of nitrogen and carbon dioxide; sulfur hexafluoride; inert gases; alkanes, partially fluorinated alkanes, perfluoroalkanes, bromofluoroalkanes in linear, branched, cyclic form; physiologically acceptable nitrogen oxides such as NO.

33. Process for producing an implant according to claim 1, **characterized in that**, during the preparation of the ultrasonically detectable elements, structural material of the ultrasonically detectable elements is expanded with the addition of a chemical or physical blowing agent, the blowing agent preferably containing at least one of the substances selected from the following group: water; non-, partially and perfluorinated n-, iso-, neo- and cycloalkanes; hydrogen phosphate/hydrogen carbonate/starch mixtures; ammonium nitrite; calcium carbonate; ammonium carbonate; mixtures of carbonate and solid acids; readily gas-eliminating monomers, oligomers and polymers, in particular maleic acid as well as its esters and anhydrides, oxocarboxylic acids, aceto acetic acid and its derivatives, á-ketopropionic acid, acetondicarboxylic acid, tartaric acid as well as the esters and salts thereof, oxalates, Diels-Alder adduct analogs from dienes with carbon dioxide or nitrogen, 3,6-dihydro-2H-pyran-2-on [26677-08-7], homo and copolymers of itaconic acid and the esters thereof, copolymers of azobisisobutyric acid with diols, ethylene glycol, diethylene glycol, triethylene glycol, tetraethylene glycol, oligoethylene glycol, polyethylene glycol, propanediol, butanediol, hexanediol; azo compounds, in particular azodicarbonamide and modified azodicarbonamide; hydrazine derivatives, in particular 4, 4' -oxybis [benzol] sulfonhydrazide, diphenylsulfon-3,3'-disulfonhydrazide, diphenylene oxide-4,4-disulfonhydrazide, trihydrazinotriazine; semicarbazides, in particular p-toluylenesulfonyl semicarbazide; tetrazoles, in particular 5-phenyltetrazole; benzooxazines, in particular isatoic acid anhydride.

34. Process for producing an implant according to claim 1, **characterized in that**, during the preparation of the ultrasonically detectable elements, an open-pored foam is gassed and then closed by thermal filming at the surface, gases with a poor blood and polymer solubility preferably being used, in particular at least one of the gases selected from the following group: perfluoromethane, perfluoroethane, perfluoropropanes, perfluorobutanes, perfluoropentanes.

35. Process for producing an implant according to claim 1, **characterized in that**, during the preparation of the ultrasonically detectable elements, an open-pored foam is gassed and then sealed by coating with a biocompatible soft powder, the powder preferably containing at least one of the substances selected from the following group: fats, waxes, readily-melting polymers, and a thermal filming preferably then being carried out.

36. Process for producing an implant according to claim 1, **characterized in that**, during the preparation of the ultrasonically detectable elements, an open-pored foam is gassed and sealed with a coating material dissolved in a solvent, the solvent then being evaporated.

37. Process for producing an implant according to claim 1, **characterized in that**, during the manufacture of the ultrasonically detectable elements, an open-pored foam is gassed and then sealed with a monomer coating, the coating then being polymerized or cross-linked.

38. Process for producing an implant according to claim 1, **characterized in that**, during the preparation of the ultrasonically detectable elements, a syntactic foam is extruded, preferably made of polypropylene, gas-filled glass hollow balls preferably being embedded in the syntactic foam.

39. Process for producing an implant according to claim 1, **characterized in that**, during the preparation of the ultrasonically detectable elements, a polymer is precipitated from a solvent in the presence of gas-containing or gas-producing microcapsules.

40. Process for producing an implant according to claim 1, **characterized in that**, during the preparation of the ultrasonically detectable elements, an interfacial polymerization with gas-containing or gas-producing microcapsules is carried out.

41. Process for producing an implant according to claim 1, **characterized in that**, during the preparation of the ultrasonically detectable elements, in the presence of gas-containing or gas-producing microcapsules a polymerization or polyaddition or polycondensation of at least one hydrophilic monomer or polymer and a chemical cross-linker is carried out.

42. Process according to claim 41, **characterized in that** the hydrogel obtained upon the polymerization or polyaddition or polycondensation contains at least one of the substances selected from the following group: polymerized hydroxyethyl methacrylate (HEMA) ; polymerized hydroxypropyl methacrylate (HPMA); polymerized α-methacryloyl-ω-methoxy polyethylene glycol; polymerized polyethylene glycol-bisacrylate; resorbable prepolymers of type A-B-C-B-A with A = acryl or methacryl groups, B = hydrolytically splittable and containing polymers of lactide, glycolide, 2-hydroxybutyric acid, 2-hydroxyvaleriac acid, trimethylene carbonate, polyorthoesters, polyanhydrides, polyphosphates, polyphosphazenes and/or polyamides and/or copolymers thereof, and C = hydrophilic polymers, in particular polyethylene glycol (PEG), polyvinyl alcohol (PVA), polyvinyl pyrrolidone (PVP), poly-N-isoprolyacrylamide (PNiPAAM).

43. Process for producing an implant according to claim 1, **characterized in that**, during the preparation of the ultrasonically detectable elements, a hollow thread or a thin tube is thermally sealed at least at both ends.

44. Process for producing an implant according to claim 1, **characterized in that**, during the preparation of the ultrasonically detectable elements, a hollow thread or a thin tube is sealed by ultrasound at least at both ends and preferably then coated with a sealing made from wax and/or polymer.

45. Process according to claim 43 or 44, **characterized in that**, before the sealing, an agent is poured into the hollow thread or thin tube which releases a gas upon the introduction of humidity, the agent preferably containing one of the following substances: baking powder, benzoic acid/carbonate mixtures, carbides, metal hydrides.

46. Process according to claim 43 or 44, **characterized in that**, before the sealing, a suspension of gas-containing or gas-producing microcapsules is poured into the hollow thread or thin tube.

47. Process according to claim 46, **characterized in that** decomposition inhibitors for the microcapsules are additionally poured into the hollow thread or thin tube before the sealing, the decomposition inhibitors preferably containing at least one of the following substances: buffers, acids, bases, polymers which set the pH value by decomposition or solvation, esterase inhibitors, protease inhibitors, dextranase inhibitors, mixed-function oxidase inhibitors, cryoprotectors or enzymes which favour the decomposition of enzymes which break down microcapsules, competitively decomposable polymers not detectable in ultrasound.

48. Process according to claim 43 or 44, **characterized in that**, before the sealing, a suspension of gas-containing or gas-producing microcapsules in dry form is poured into the hollow thread or thin tube.

49. Process according to claim 43 or 44, **characterized in that**, before the sealing, an emulsion which vaporizes under diagnostic ultrasound is poured into the hollow thread or thin tube.

50. Process for producing an implant according to claim 1, **characterized in that**, for the gentle preparation of the ultrasonically detectable elements, gas-filled microparticles are encapsulated in a hydrogel below 50°C in the presence of hydrophilic mono- or bifunctional monomers and/or polymers.

51. Process for producing an implant according to claim 1, **characterized in that**, for the gentle preparation of the ultrasonically detectable elements, gas-filled microparticles are encapsulated below 50°C in the presence of a dialdehyde and a polyamine or protein.

52. Process according to claim 50 or 51, **characterized in that** the encapsulation is carried out directly on the implant.

53. Process according to claim 50 or 51, **characterized in that** the encapsulation is carried out in the form of pre-shaped bodies or linear structures, in particular threads, and the pre-shaped bodies or linear structures are attached to the flexible basic structure of the implant after the encapsulation.

54. Process for producing an implant according to claim 1, **characterized in that**, during the preparation of the ultrasonically detectable elements, gas-filled microcapsules or their suspensions are used as preliminary steps to the in-situ generation of bubbles in pre-shaped bodies or linear structures, in particular threads.

55. Process for producing an implant according to claim 1, **characterized in that** echogenic microcapsules are used as precursors for generating bubbles in the implant.

## Patentansprüche

1. Flächiges Implantat, mit einer flexiblen Grundstruktur (1; 30, 32; 40, 42; 50) auf Polymerbasis und mit im Ultraschall detektierbaren Elementen (2; 10; 20; 32; 42; 52), die gashaltig bzw. gaserzeugend sind und die für eine Detektierbarkeit nach Implantation von mindestens vier Wochen eingerichtet sind.

2. Implantat nach Anspruch 1, **dadurch gekennzeichnet, dass** die im Ultraschall detektierbaren Elemente (52) in einem flächigen Muster angeordnet sind.

3. Implantat nach Anspruch 1 oder 2, **dadurch gekennzeichnet, dass** die Grundstruktur (1; 30; 40) nichtresorbierbares Polymer aufweist.

4. Implantat nach Anspruch 3, **dadurch gekennzeichnet, dass** die Grundstruktur (1; 30; 40) mindestens eine der aus der folgenden Gruppe ausgewählten Substanzen aufweist: Polyalkene, Polypropylen, Polyethylen, teilhalogenisierte Polyolefine, vollhalogenisierte Polyolefine, fluorierte Polyolefine, Polytetrafluorethylen, Polyvinylidenfluorid, Polyisoprene, Polystyrole, Polysilikone, Polycarbonate, Polyaryletherketone, Polymethacrylsäureester, Polyacrylsäureester, Polyimide, Copolymere von polymerisierbaren Substanzen davon.

5. Implantat nach einem der Ansprüche 1 bis 4., **dadurch gekennzeichnet, dass** die Grundstruktur (50) resorbierbares Polymer aufweist.

6. Implantat nach Anspruch 5, **dadurch gekennzeichnet, dass** die Grundstruktur (50) mindestens eine der aus der folgenden Gruppe ausgewählten Substanzen aufweist: Polyhydroxysäuren, Polylactide, Polyglykolide, Polyhydroxybutyrate, Polyhydroxyvaleriate, Polycaprolactone, Polydioxanone, synthetische und natürliche Oligo- und Polyaminosäuren, Polyphosphazene, Polyanhydride, Polyorthoester, Polyphosphate, Polyphosphonate, Polyalkohole, Polyzucker, Polyether, Polyamide, aliphatische Polyester, aromatische Polyester, Copolymere von polymerisierbaren Substanzen davon, resorbierbare Gläser.

7. Implantat nach einem der Ansprüche 1 bis 6, **dadurch gekennzeichnet, dass** die Grundstruktur eine der aus der folgenden Gruppe ausgewählten Formen hat: Netze (1), Bänder (50), Folien, gelochte Folien, Filze, Vliese, offenporige Schaumfolien.

8. Implantat nach einem der Ansprüche 1 bis 7, **dadurch gekennzeichnet, dass** die im Ultraschall detektierbaren Elemente (2) mindestens eine der aus der folgenden Gruppe ausgewählten Substanzen aufweisen: physiologisch vertretbare Gase; leichtsiedende Flüssigkeiten, die bei 38°C gasförmig vorliegen; leichtsiedende Flüssigkeiten, die im Ultraschallfeld aufgasen.

9. Implantat nach Anspruch 8, **dadurch gekennzeichnet, dass** die im Ultraschall detektierbaren Elemente (2; 10; 20; 32; 42; 52) mindestens eine der aus der folgenden Gruppe ausgewählten Substanzen aufweisen: nicht-, teil- und perfluorierte n-, iso-, neo- und Cycloalkane, Fluorbromalkane, Schwefelhexafluorid, Wasserstoff, Stickstoff, Sauerstoff, Luft, Kohlendioxid, Helium, Neon, Argon, Xenon, Krypton.

10. Implantat nach Anspruch einem der Ansprüche 1 bis 9, **dadurch gekennzeichnet, dass** die im Ultraschall detektierbaren Elemente (2; 10; 20; 32; 42) ein nichtresorbierbares Strukturmaterial aufweisen.

11. Implantat nach Anspruch 10, **dadurch gekennzeichnet, dass** das Strukturmaterial der im Ultraschall detektierbaren Elemente (2; 10; 20; 32; 42) mindestens eine der aus der folgenden Gruppe ausgewählten Substanzen aufweist: Polyalkene, Polypropylen, Polyethylen, teilhalogenisierte Polyolefine, vollhalogenisierte Polyolefine, fluorierte Polyolefine, Polytetrafluorethylen, Polyvinylidenfluorid, Polyisoprene, Polystyrole, Polysilikone, Polycarbonate, Polyaryletherketone, Polymethacrylsäureester, Polyacrylsäureester, Polyimide, hydrophile quervernetzte Polymere, Silikone, Copolymere von polymerisierbaren Substanzen davon, Keramiken, Gläser, Metalle, Kohlenstoff.

12. Implantat nach Anspruch einem der Ansprüche 1 bis 11, **dadurch gekennzeichnet, dass** die im Ultraschall detektierbaren Elemente (52) ein resorbierbares Strukturmaterial aufweisen.

13. Implantat nach Anspruch 12, **dadurch gekennzeichnet, dass** das Strukturmaterial der im Ultraschall detektierbaren Elemente (52) mindestens eine der aus der folgenden Gruppe ausgewählten Substanzen aufweist: Polyhydroxysäuren, Polylactide, Polyglykolide, Polyhydroxybutyrate, Polyhydroxyvaleriate, Polycaprolactone, Polydioxanone, synthetische und natürliche Oligo- und Polyaminosäuren, Polyphosphazene, Polyanhydride, Polyorthoester, Polyphosphate, Polyphosphonate, Polyalkohole, Polyzucker, Polyether, Polyamide, aliphatische Polyester, aromatische Polyester, natürliche Polyaminosäuren, synthetische Polyaminosäuren, gentechnisch erzeugte Polyaminosäuren, Collagen, rhCollagen, Seide, Pseudopolyaminosäuren, Polycyanacrylate, Polyethylenglycole, Polyvinylalkohole, derivatisierte Zellulose, Fette, Wachse, Fettsäuren, Fettsäureester, Polyphosphatester, Copolymere von polymerisierbaren Substanzen davon, resorbierbare Gläser.

14. Implantat nach einem der Ansprüche 1 bis 13, **dadurch gekennzeichnet, dass** zumindest ein Teil der im Ultraschall detektierbaren Elemente als Formkörper (2; 10; 52) mit jeweiliger Länge, Breite und Höhe im Bereich von 0,1 mm bis 50 mm ausgebildet ist.

15. Implantat nach Anspruch 14, **dadurch gekennzeichnet, dass** zumindest ein Formkörper eine der aus der folgenden Gruppe ausgewählten Formen hat: Ringe (2), Scheiben, Plättchen, Knöpfe, flächige Ellipsen, Halbkugeln, Vollkugeln, Perlen, Zylinder, Würfel, Quader, Kegel, Stäbe, Hülsen, Schläuche (10), Röhrchen, Folien (52).

16. Implantat nach einem der Ansprüche 1 bis 15, **dadurch gekennzeichnet, dass** zumindest ein Teil der im Ultraschall detektierbaren Elemente als Lineargebilde (20; 32; 42) ausgestaltet ist.

17. Implantat nach Anspruch 16, **dadurch gekennzeichnet, dass** zumindest ein Lineargebilde von einem aus der folgenden Gruppe ausgewählten Typ ist: Bänder, Kordeln, Fäden (20; 40), Zwirne, geknotete Filamente, Folienbändchen (32), Umwindezwirne.

18. Implantat nach einem der Ansprüche 1 bis 17, **dadurch gekennzeichnet, dass** zumindest ein Teil der im Ultraschall detektierbaren Elemente auf mindestens eine der aus der folgenden Gruppe ausgewählten Arten mit der Grundstruktur verbunden ist: Aufschmelzen, Verschweißen (2; 10), Aufbringen aus Lösung (52), Verkleben, Verknoten, Befestigung an einer mit der Grundstruktur verbundenen Halteeinrichtung, Einarbeitung in die Grundstruktur durch textile Techniken (32; 42).

19. Implantat nach einem der Ansprüche 1 bis 18, **dadurch gekennzeichnet, dass** zumindest ein Teil der im Ultraschall detektierbaren Elemente ein im Ultraschall detektierbares Symbol aufweist, das vorzugsweise mehrfach und in gleichem Abstand vorgesehen ist.

20. Implantat nach Anspruch 19, **dadurch gekennzeichnet, dass** das Symbol in einer aus der folgenden Gruppe ausgewählten Formen ausgestaltet ist: aus Lineargebilden genäht, aus Lineargebilden gestickt, aus Folie geprägt, aus mehreren Objekten zusammengesetzt.

21. Implantat nach einem der Ansprüche 1 bis 20, **dadurch gekennzeichnet, dass** zumindest ein Teil der im Ultraschall detektierbaren Elemente (2) eine Struktur mit einem einbis vielzelligen Integralschaum aufweist, dessen Innenwände trocken oder mit einer Flüssigkeit benetzt sind.

22. Implantat nach einem der Ansprüche 1 bis 21, **dadurch gekennzeichnet, dass** zumindest ein Teil der im Ultraschall detektierbaren Elemente (20; 32; 42) eine Matrix aufweist, in die gasgefüllte Mikrokapseln (22) eingebettet sind.

23. Implantat nach Anspruch 22, **dadurch gekennzeichnet, dass** die Mikrokapseln auf mindestens eine der aus der folgenden Gruppe ausgewählten Arten vorliegen: von Flüssigkeit umgebene Mikrokapseln, die in Polymer eingebettet sind; von Flüssigkeit umgebene Mikrokapseln, die in Fett eingebettet sind; von Flüssigkeit umgebene Mikrokapseln, die in ein Organogel eingebettet sind; von Flüssigkeit umgebene Mikrokapseln, die in ein Glas eingebettet sind; Mikrokapseln (22), die in ein hydrophobes Polymer eingebettet sind; Mikrokapseln, die in ein hydrophiles Gel eingebettet sind; Mikrokapseln, die in ein quervernetztes Polymer eingebettet sind; Mikrokapseln, die in ein Polymergel eingebettet sind; Mikrokapseln, die in einen offenporigen Polymerschaum eingebettet sind, wobei der Durchmesser der Mikrokapseln generell größer ist als der Porendurchmesser der äußeren Schaumporen.

24. Implantat nach einem der Ansprüche 1 bis 23, **dadurch gekennzeichnet, dass** zumindest ein Teil der im Ultraschall detektierbaren Elemente eine Struktur mit einem Komposit, das Hohlfäden in einer Polymermatrix enthält, aufweist.

25. Implantat nach einem der Ansprüche 1 bis 24, **dadurch gekennzeichnet, dass** zumindest ein Teil der im Ultraschall detektierbaren Elemente eine Struktur mit offenporigen Formkörpern bzw. Lineargebilden aufweist, die von einer gasdichten Hülle umgeben sind.

26. Implantat nach einem der Ansprüche 1 bis 25, **dadurch gekennzeichnet, dass** zumindest ein Teil der im Ultraschall detektierbaren Elemente zumindest teilweise aus gasgefüllten Mikropartikeln zusammengesetzt ist, wobei die Mikropartikeln oberflächlich fusioniert sind, und zwar vorzugsweise oberflächlich thermisch verfilmt, ionisch vernetzt und/oder chemisch vernetzt.

27. Implantat nach einem der Ansprüche 1 bis 26, **dadurch gekennzeichnet, dass** zumindest ein Teil der im Ultraschall detektierbaren Elemente (52) eine Struktur mit Blasenfolien aufweist.

28. Implantat nach einem der Ansprüche 1 bis 27, **dadurch gekennzeichnet, dass** das Implantat auch in magnetischer Resonanztomographie detektierbar ist.

29. Implantat nach Anspruch 28, **gekennzeichnet durch** Formkörper bzw. Lineargebilde, die einen Magnetresonanzkontrast begünstigen, wobei die Formkörper bzw. Lineargebilde vorzugsweise mit Wasser, Magnetresonanz-Kontrastmittel und/oder Fett gefüllte Schläuche aufweisen.

30. Implantat nach einem der Ansprüche 1 bis 29, **gekennzeichnet durch** mindestens einen biologisch aktiven Wirkstoff, der vorzugsweise mindestens eine der aus der folgenden Gruppe ausgewählten Substanzen aufweist: natürliche Wirkstoffe, synthetische Wirkstoffe, Antibiotika, Chemotherapeutika, Zytostatika, Metastasehemmer, Antidiabetika, Antimykotika, Gynekologika, Urologika, Antiallergika, Sexualhormone, Hemmstoffe von Sexualhormonen, Hämostyptika, Hormone, Peptidhormone, Antidepressiva, Antihistaminika, nackte DNA, Plasmid-DNA, kationische DNA-Komplexe, RNA, Zellbestandteile, Impfstoffe, körpereigene Zellen, gentechnisch modifizierte Zellen.

31. Implantat nach Anspruch 30, **dadurch gekennzeichnet, dass** der Wirkstoff in mindestens einer der aus der folgenden Gruppe ausgewählten Formen vorliegt: in verkapselter Form, in adsorbierter Form, in der Grundstruktur, an der Grundstruktur, in im Ultraschall detektierbaren Elementen, an im Ultraschall detektierbaren Elementen.

32. Verfahren zum Herstellen eines Implantats nach Anspruch 1, **dadurch gekennzeichnet, dass** beim Herstellen der im Ultraschall detektierbaren Elemente Strukturmaterial der im Ultraschall detektierbaren Elemente extrudiert wird und dabei ein Gas durch Direktbegasung oder unter überkritischen Bedingungen in das Strukturmaterial eingebracht wird, wobei das Gas vorzugsweise mindestens eine der aus der folgenden Gruppe ausgewählten Substanzen aufweist: Luft, Kohlendioxid, Stickstoff, Gemische aus Stickstoff und Kohlendioxid; Schwefelhexafluorid; Edelgase; Alkane, teilfluorierte Alkane, Perfluoralkane, Bromfluoralkane, in linearer, verzweigter, cyclischer Form; physiologisch vertretbare Stickoxide wie NO.

33. Verfahren zum Herstellen eines Implantats nach Anspruch 1, **dadurch gekennzeichnet, dass** beim Herstellen der im Ultraschall detektierbaren Elemente Strukturmaterial der im Ultraschall detektierbaren Elemente unter Zugabe eines chemischen bzw. physikalischen Treibmittels expandiert wird, wobei das Treibmittel vorzugsweise mindestens eine der aus der folgenden Gruppe ausgewählten Substanzen aufweist: Wasser; nicht-, teil- und perfluorierte n-, iso-, neo- und Cycloalkane; Hydrogenphosphat/Hydrogencarbonat/-Stärke-Mischungen; Ammoniumnitrit; Calciumcarbonat; Ammoniumcarbonat; Mischungen aus Carbonat und festen Säuren; leicht gaseliminierende Monomere, Oligomere und Polymere, insbesondere Maleinsäure sowie deren Ester und Anhydride, Oxocarbonsäuren, Acetessigsäure und deren Derivate, α-Ketopropionsäure, Acetondicarbonsäure, Weinsäure sowie deren Ester und Salze, Oxalate, Diels-Alder-Addukt-Analoge aus Dienen mit Kohlendioxid bzw. Stickstoff, 3,6-Dihydro-2H-pyran-2-on [26677-08-7], Homo- und Copolymere der Itaconconsäure und deren Ester, Copolymere aus Azo-bis-isobuttersäure mit Diolen, Ethylenglycol, Diethylenglycol, Triethylenglycol, Tetraethylenglycol, Oligoethylenglycol, Polyethylenglycol, Propandiol, Butandiol, Hexandiol; Azoverbindungen, insbesondere Azodicarbonamid und modifiziertes Azodicarbonamid; Hydrazin-Derivate, insbesondere 4,4'-Oxybis[benzol]sulfonhydrazid,Diphenylsulfon-3,3'-disulfon-hydrazid, Diphenylenoxid-4,4-disulfonhydrazid, Trihydrazinotriazin; Semicarbazide, insbesondere p-Toluylensulfonylsemicarbazid; Tetrazole, insbesondere 5-Phenyltetrazol; Benzooxazine, insbesondere Isatosäureanhydrid.

34. Verfahren zum Herstellen eines Implantats nach Anspruch 1, **dadurch gekennzeichnet, dass** beim Herstellen der im Ultraschall detektierbaren Elemente ein offenporiger Schaum begast und anschließend durch thermische Verfilmung an der Oberfläche geschlossen wird, wobei als Gas vorzugsweise Gase mit einer schlechten Blut- und Polymerlöslichkeit verwendet werden, insbesondere mindestens ein aus der folgenden Gruppe ausgewähltes Gas: Perfluormethan, Perfluorethan, Perfluorpropane, Perfluorbutane, Perfluorpentane.

35. Verfahren zum Herstellen eines Implantats nach Anspruch 1, **dadurch gekennzeichnet, dass** beim Herstellen der im Ultraschall detektierbaren Elemente ein offenporiger Schaum begast und anschließend durch Beschichten mit einem biokompatiblen weichen Puder verschlossen wird, wobei der Puder vorzugsweise mindestens eine der aus der folgenden Gruppe ausgewählten Substanzen aufweist: Fette, Wachse, leichtschmelzende Polymere, und wobei vorzugsweise anschließend eine thermische Verfilmung durchgeführt wird.

36. Verfahren zum Herstellen eines Implantats nach Anspruch 1, **dadurch gekennzeichnet, dass** beim Herstellen der im Ultraschall detektierbaren Elemente ein offenporiger Schaum begast und mit einem in einem Lösungsmittel gelösten Beschichtungsmaterial verschlossen wird, wobei das Lösungsmittel anschließend verdampft wird.

37. Verfahren zum Herstellen eines Implantats nach Anspruch 1, **dadurch gekennzeichnet, dass** beim Herstellen der im Ultraschall detektierbaren Elemente ein offenporiger Schaum begast und danach mit einer Monomerbeschichtung verschlossen wird, wobei die Beschichtung anschließend polymerisiert bzw. quervernetzt wird.

38. Verfahren zum Herstellen eines Implantats nach Anspruch 1, **dadurch gekennzeichnet, dass** beim Herstellen der im Ultraschall detektierbaren Elemente ein syntaktischer Schaum extrudiert wird, vorzugsweise aus Polypropylen, wobei vorzugsweise gasgefüllte Glashohlkugeln in den syntaktischen Schaum eingebettet werden.

39. Verfahren zum Herstellen eines Implantats nach Anspruch 1, **dadurch gekennzeichnet, dass** beim Herstellen der im Ultraschall detektierbaren Elemente ein Polymer in Gegenwart von gashaltigen bzw. gaserzeugenden Mikrokapseln aus einem Lösungsmittel gefällt wird.

40. Verfahren zum Herstellen eines Implantats nach Anspruch 1, **dadurch gekennzeichnet, dass** beim Herstellen der im Ultraschall detektierbaren Elemente eine Grenzflächenpolymerisation mit gashaltigen bzw. gaserzeugenden Mikrokapseln durchgeführt wird.

41. Verfahren zum Herstellen eines Implantats nach Anspruch 1, **dadurch gekennzeichnet, dass** beim Herstellen der im Ultraschall detektierbaren Elemente in Gegenwart gashaltiger bzw. gaserzeugender Mikrokapseln eine Polymerisation bzw. Polyaddition bzw. Polykoridensation von mindestens einem hydrophilen Monomer bzw. Polymer und einem chemischen Quervernetzer durchgeführt wird.

42. Verfahren nach Anspruch 41, **dadurch gekennzeichnet, dass** das bei der Polymerisation bzw. Polyaddition bzw. Polykondensation erhaltene Hydrogel mindestens eine der aus der folgenden Gruppe ausgewählten Substanzen aufweist: polymerisiertes Hydroxyethylmethacrylat (HEMA); polymerisiertes Hydroxypropylmethacrylat (HPMA); polymerisiertes α-Methacryloyl-ω-Methoxypolyethylenglycol; polymerisiertes Polyethylenglycol-bis-acrylat; resorbierbare Prepolymere vom Typ A-B-C-B-A, mit A = Acryl- oder Methacrylgruppen, B = hydrolytisch spaltbar und enthaltend Polymere aus Lactid, Glykolid, 2-Hydroxybuttersäure, 2-Hydroxyvaleriansäure, Trimethylencarbonat, Polyorthoester, Polyanhydriden, Polyphospaten, Polyphosphazenen und/oder Polyamiden und/oder Copolymere daraus, und C = hydrophile Polymere, insbesondere Polyethylenglykol (PEG), Polyvinylalkohol (PVA), Polyvinylpyrrolidon (PVP), Poly-N-isoprolyacrylamid (PNiPAAM).

43. Verfahren zum Herstellen eines Implantats nach Anspruch 1, **dadurch gekennzeichnet, dass** beim Herstellen der im Ultraschall detektierbaren Elemente ein Hohlfaden oder ein dünner Schlauch zumindest an beiden Enden thermisch verschmolzen wird.

44. Verfahren zum Herstellen eines Implantats nach Anspruch 1, **dadurch gekennzeichnet, dass** beim Herstellen der im Ultraschall detektierbaren Elemente ein Hohlfaden oder ein dünner Schlauch zumindest an beiden Enden durch Ultraschall verschmolzen wird und vorzugsweise danach mit einer Versiegelung aus Wachs und/oder Polymer beschichtet wird.

45. Verfahren nach Anspruch 43 oder 44, **dadurch gekennzeichnet, dass** in den Hohlfaden oder dünnen Schlauch vor dem Verschmelzen ein Mittel eingefüllt wird, das bei Feuchtigkeitseintritt ein Gas freisetzt, wobei das Mittel vorzugsweise mindestens eine der folgenden Substanzen aufweist: Backpulver, Benzoesäure/Carbonat-Mischungen, Carbide, Metallhydride.

46. Verfahren nach Anspruch 43 oder 44, **dadurch gekennzeichnet, dass** in den Hohlfaden oder dünnen Schlauch vor dem Verschmelzen eine Suspension von gashaltigen bzw. gaserzeugenden Mikrokapseln eingefüllt wird.

47. Verfahren nach Anspruch 46, **dadurch gekennzeichnet, dass** in den Hohlfaden oder dünnen Schlauch vor dem Verschmelzen zusätzlich Abbauinhibitoren für die Mikrokapseln eingefüllt werden, wobei die Abbauinhibitoren vorzugsweise mindestens eine der folgenden Substanzen aufweisen: Puffer, Säuren, Basen, durch Abbau oder Solvatation den pH-Wert einstellende Polymere, Esterasehemmer, Proteasehemmer, Dextranasehemmer, Mischfunktionelloxidase-Hemmer, den Abbau von Mikrokapseln abbauenden Enzymen begünstigende Kryoprotektoren bzw. Enzyme, nicht im Ultraschall detektierbare kompetitiv abbaubare Polymere.

48. Verfahren nach Anspruch 43 oder 44, **dadurch gekennzeichnet, dass** in den Hohlfaden oder dünnen Schlauch vor dem Verschmelzen eine Suspension von gashaltigen bzw. gaserzeugenden Mikrokapseln in trockener Form eingefüllt wird.

49. Verfahren nach Anspruch 43 oder 44, **dadurch gekennzeichnet, dass** in den Hohlfaden oder dünnen Schlauch vor dem Verschmelzen eine unter diagnostischem Ultraschall aufgasende Emulsion eingefüllt wird.

50. Verfahren zum Herstellen eines Implantats nach Anspruch 1, **dadurch gekennzeichnet, dass** zur schonenden Herstellung der im Ultraschall detektierbaren Elemente gasgefüllte Mikropartikel unterhalb von 50°C in Gegenwart von hydrophilen mono- oder difunktionellen Monomeren und/oder Polymeren in einem Hydrogel verkapselt werden.

51. Verfahren zum Herstellen eines Implantats nach Anspruch 1, **dadurch gekennzeichnet, dass** zur schonenden Herstellung der im Ultraschall detektierbaren Elemente gasgefüllte Mikropartikel unterhalb von 50°C in Gegenwart eines Dialdehyds und eines Polyamins oder Proteins verkapselt werden.

52. Verfahren nach Anspruch 50 oder 51, **dadurch gekennzeichnet, dass** die Verkapselung direkt auf dem Implantat erfolgt.

53. Verfahren nach Anspruch 50 oder 51, **dadurch gekennzeichnet, dass** die Verkapselung in Form von Formkörpern oder Lineargebilden, insbesondere Fäden, erfolgt und die Formkörper bzw. Lineargebilde nach der Verkapselung an der flexiblen Grundstruktur des Implantats befestigt werden.

54. Verfahren zum Herstellen eines Implantats nach Anspruch 1, **dadurch gekennzeichnet, dass** beim Herstellen der im Ultraschall detektierbaren Elemente gasgefüllte Mikrokapseln oder deren Suspensionen als Vorstufen zur in-situ Erzeugung von Blasen in Formkörpern oder Lineargebilden, insbesondere Fäden, verwendet werden.

55. Verfahren zum Herstellen eines Implantats nach Anspruch 1, **dadurch gekennzeichnet, dass** echogene Mikrokapseln als Ausgangsteilchen zur Herstellung von Blasen im Implantat verwendet werden.

## Revendications

1. Implant aéral, avec une structure de base flexible sur une base polymère (1 ; 30, 32 ; 40, 42 ; 50) et avec des éléments détectables par ultrasons (2 ; 10 ; 20 ; 32 ; 42 ; 52), qui contiennent ou produisent un gaz et qui sont adaptés pour être détectables pendant au moins quatre semaines après l'implantation.

2. Implant selon la revendication 1, **caractérisé en ce que** les éléments (52) détectables par ultrasons sont agencés dans un motif aéral.

3. Implant selon la revendication 1 ou 2, **caractérisé en ce que** la structure de base (1 ; 30 ; 40) contient un polymère non résorbable.

4. Implant selon la revendication 3, **caractérisé en ce que** la structure de base (1 ; 30 ; 40) contient au moins l'une des substances choisies dans le groupe suivant : les polyalcènes, le polypropylène, le polyéthylène, les polyoléfines partiellement halogénées, les polyoléfines entièrement halogénées, les polyoléfines fluorées, le polytétrafluoréthylène, le fluorure de polyvinylidène, les polyisoprènes, les polystyrènes, les polysilicones, les polycarbonates, les polyaryléther cétones, les esters d'acide polyméthacrylique, les esters d'acide polyacrylique, les polyimides, les copolymères de substances polymérisables de ces composés.

5. Implant selon l'une des revendications 1 à 4, **caractérisé en ce que** la structure de base (50) contient un polymère résorbable.

6. Implant selon la revendication 5, **caractérisé en ce que** la structure de base (50) contient au moins l'une des substances choisies dans le groupe suivant : les polyhydroxy acides, les polylactides, les polyglycolides, les polyhydroxy butyrates, les polyhydroxy valériates, les polycaprolactones, les polydioxanones, les oligo- et polyamino acides synthétiques et naturels, les polyphosphazènes, les polyanhydrides, les polyorthoesters, les polyphosphates, les polyphosphonates, les polyalcools, les polysaccharides, les polyéthers, les polyamides, les polyesters aliphatiques, les polyesters aromatiques, les copolymères de substances polymérisables de ces composés, les verres résorbables.

7. Implant selon l'une des revendications 1 à 6, **caractérisé en ce que** la structure de base a l'une des formes choisies dans le groupe suivant: mailles (1), bandes (50), films, films perforés, feutres, molletons, films mousses à pores ouverts.

8. Implant selon l'une des revendications 1 à 7, **caractérisé en ce que** les éléments (2) détectables par ultrasons contiennent au moins l'une des substances choisies dans le groupe suivant : les gaz physiologiquement acceptables ; les liquides à point d'ébullition bas qui sont présents sous forme gazeuse à 38 °C ; les liquides à point d'ébullition bas qui s'évaporent dans la gamme des ultrasons.

9. Implant selon la revendication 8, **caractérisé en ce que** les éléments (2 ; 10 ; 20 ; 32 ; 42 ; 52) détectables par ultrasons contiennent au moins l'une des substances choisies dans le groupe suivant : les n-, iso-, néo- et cycloalcanes non-, partiellement et perfluorés, les fluorobromoalcanes, l'hexafluorure de soufre, l'hydrogène, l'azote, l'oxygène, l'air, le dioxyde de carbone, l'hélium, le néon, l'argon, le xénon, le krypton.

10. Implant selon l'une des revendications 1 à 9, **caractérisé en ce que** les éléments (2 ; 10 ; 20 ; 32 ; 42) détectables par ultrasons contiennent un matériau structural non résorbable.

11. Implant selon la revendication 10, **caractérisé en ce que** le matériau structural des éléments (2 ; 10 ; 20 ; 32 ; 42) détectables par ultrasons contient au moins l'une des substances choisies dans le groupe suivant : les polyalcènes, le polypropylène, le polyéthylène, les polyoléfines partiellement halogénées, les polyoléfines entièrement halogénées, les polyoléfines fluorées, le polytétrafluoréthylène, le fluorure de polyvinylidène, les polyisoprènes, les polystyrènes, les polysilicones, les polycarbonates, les polyaryléther cétones, les esters d'acide polyméthacrylique, les esters d'acide polyacrylique, les polyimides, les polymères réticulés hydrophiles, les silicones, les copolymères de substances polymérisables de ces composés, les céramiques, les verres, les métaux, le carbone.

12. Implant selon l'une des revendications 1 à 11, **caractérisé en ce que** les éléments (52) détectables par ultrasons contiennent un matériau structural résorbable.

13. Implant selon la revendication 12, **caractérisé en ce que** le matériau structural des éléments (52) détectables par ultrasons contient au moins l'une des substances choisies dans le groupe suivant : les polyhydroxy acides, les polylactides, les polyglycolides, les polyhydroxybutyrates, les polyhydroxyvalériates, les polycaprolactones, les polydioxanones, les oligo- et polyamino acides synthétiques et naturels, les polyphosphazènes, les polyanhydrides, les polyorthoesters, les polyphosphates, les polyphosphonates, les polyalcools, les polysaccharides, les polyéthers, les polyamides, les polyesters aliphatiques, les polyesters aromatiques, les polyamino acides naturels, les polyamino acides synthétiques, les polyamino acides produits génétiquement, le collagène, le rhcollagène, la soie, les pseudo-polyamino acides, les polycyanoacrylates, les polyéthylène glycols, les polyvinyl alcools, la cellulose dérivée, les graisses, les cires, les acides gras, les esters d'acides gras, les esters de polyphosphate, les copolymères de substances polymérisables de ces composés, les verres résorbables.

14. Implant selon l'une des revendications 1 à 13, **caractérisé en ce qu'**au moins une partie des éléments détectables par ultrasons est sous la forme de corps pré-formés (2 ; 10 ; 52) avec une longueur, une largeur et une hauteur respectives dans l'intervalle de 0,1 mm à 50 mm.

15. Implant selon la revendication 14, **caractérisé en ce qu'**au moins un corps pré-formé a l'une des formes choisies dans le groupe suivant : anneaux (2), disques, plaquettes, boutons, ellipses plates, hémisphères, sphères solides, perles, cylindres, cubes, blocs, cônes, tiges, manchons, tubes (10), tuyaux, films (52).

16. Implant selon l'une des revendications 1 à 15, **caractérisé en ce qu'**au moins une partie des éléments détectables par ultrasons est conçue sous la forme de structures linéaires (20 ; 32 ; 42).

17. Implant selon la revendication 16, **caractérisé en ce qu'**au moins une structure linéaire est d'un type choisi dans le groupe suivant : bandes, cordes, fils (20 ; 40), ficelles, filaments noués, bandes de films (32), ficelles de fond.

18. Implant selon l'une des revendications 1 à 17, **caractérisé en ce qu'**au moins une partie des éléments détectables par ultrasons est connectée à la structure de base selon au moins une des manières choisies dans le groupe suivant : fusion superficielle, soudure (2 ; 10), application à partir d'une solution (52), collage, nouage, fixation à un dispositif de serrage connecté à la structure de base, incorporation dans la structure de base en utilisant des techniques du textile (32 ; 42).

19. Implant selon l'une des revendications 1 à 18, **caractérisé en ce qu'**au moins une partie des éléments détectables par ultrasons a un symbole détectable par ultrasons, qui est de préférence fourni de manière répétitive à des intervalles uniformes.

20. Implant selon la revendication 19, **caractérisé en ce que** le symbole est conçu dans une des formes choisies dans le groupe suivant : cousu à partir de structures linéaires, brodé à partir de structures linéaires, moiré à partir d'un film, composé de plusieurs objets.

21. Implant selon l'une des revendications 1 à 20, **caractérisé en ce qu'**au moins une partie des éléments (2) détectables par ultrasons a une structure avec une mousse intégrale à alvéole unique ou à alvéoles multiples, dont les parois internes sont sèches ou humidifiées par un liquide.

22. Implant selon l'une des revendications 1 à 21, **caractérisé en ce qu'**au moins une partie des éléments (20 ; 32 ; 42) détectables par ultrasons contient une matrice dans laquelle des microcapsules remplies de gaz (22) sont incluses.

23. Implant selon la revendication 22, **caractérisé en ce que** les microcapsules sont présentes selon au moins une des manières choisies dans le groupe suivant : des microcapsules entourées par un liquide qui sont incluses dans un polymère ; des microcapsules entourées par un liquide qui sont incluses dans de la graisse ; des microcapsules entourées par un liquide qui sont incluses dans un organo-gel ; des microcapsules entourées par un liquide qui sont incluses dans un verre ; des microcapsules (22) qui sont incluses dans un polymère hydrophobe ; des microcapsules qui sont incluses dans un gel hydrophile ; des microcapsules qui sont incluses dans un polymère réticulé ; des microcapsules qui sont incluses dans un gel de polymère ; des microcapsules qui sont incluses dans une mousse de polymère à pores ouverts, le diamètre des microcapsules étant généralement supérieur au diamètre de pores des pores de la mousse externe.

24. Implant selon l'une des revendications 1 à 23, **caractérisé en ce qu'**au moins une partie des éléments détectables par ultrasons a une structure avec un composite qui contient des fils creux dans une matrice de polymère.

25. Implant selon l'une des revendications 1 à 2.4, **caractérisé en ce qu'**au moins une partie des éléments détectables par ultrasons a une structure avec des corps pré-formés à pores ouverts ou des structures linéaires qui sont entourées par une enveloppe étanche aux gaz.

26. Implant selon l'une des revendications 1 à 25, **caractérisé en ce qu'**au moins une partie des éléments détectables par ultrasons est composée au moins partiellement de microparticules remplies de gaz, les microparticules étant fusionnées en surface, à savoir de préférence recouvertes thermiquement d'un film superficiel, réticulées ioniquement et/ou réticulées chimiquement.

27. Implant selon l'une des revendications 1 à 26, **caractérisé en ce qu'**au moins une partie des éléments (52) détectables par ultrasons comprend une structure avec des films à cloques.

28. Implant selon l'une des revendications 1 à 27, **caractérisé en ce que** l'implant est également détectable par tomographie par résonance magnétique.

29. Implant selon la revendication 28, **caractérisé par** des corps pré-formés ou des structures linéaires qui favorisent un contraste par résonance magnétique, les corps pré-formés ou les structures linéaires comprenant de préférence des tubes remplis d'eau, d'agents de contraste par résonance magnétique et/ou de la graisse.

30. Implant selon l'une des revendications 1 à 29, **caractérisé par** au moins un ingrédient actif biologiquement qui comprend de préférence au moins l'une des substances choisies dans le groupe suivant : les ingrédients naturels, les ingrédients synthétiques, les antibiotiques, les substances chimiothérapeutiques, les substances cytostatiques, les inhibiteurs de métastase, les substances antidiabétiques, les substances anti-mycotiques, les agents gynécologiques, les agents urologiques, les agents anti-allergiques, les hormones sexuelles, les inhibiteurs des hormones sexuelles, les substances hémostatiques, les hormones, les hormones peptidiques, les antidépresseurs, les antihistaminiques, l'ADN nu, l'ADN plasmidique, les complexes d'ADN cationique, l'ARN, les constituants cellulaires, les vaccins, les cellules qui se trouvent naturellement dans le corps, les cellules génétiquement modifiées.

31. Implant selon la revendication 30, **caractérisé en ce que** l'ingrédient actif est présent dans au moins l'une des formes choisies dans le groupe suivant : dans une forme encapsulée, dans une forme adsorbée, dans la structure de base, au niveau de la structure de base, dans les éléments détectables par ultrasons, au niveau des éléments détectables par ultrasons.

32. Procédé de production d'un implant selon la revendication 1, **caractérisé en ce que**, pendant la préparation des éléments détectables par ultrasons, le matériau structural des éléments détectables par ultrasons est extrudé et, pendant ce temps, un gaz est introduit dans le matériau structural par gazage direct ou dans des conditions supercritiques, le gaz contenant de préférence au moins l'une des substances choisies dans le groupe suivant : l'air, le dioxyde de carbone, l'azote, les mélanges d'azote et de dioxyde de carbone ; l'hexafluorure de soufre ; les gaz inertes ; les alcanes, les alcanes partiellement fluorés, les perfluoroalcanes, les bromofluoroalcanes dans une forme linéaire, ramifiée, cyclique ; les oxydes d'azote physiologiquement acceptables tels que le NO.

33. Procédé de production d'un implant selon la revendication 1, **caractérisé en ce que**, pendant la préparation des éléments détectables par ultrasons, le matériau structural des éléments détectables par ultrasons est expansé avec l'addition d'un agent d'expansion chimique ou physique, l'agent d'expansion contenant de préférence au moins l'une des substances choisies dans le groupe suivant : l'eau ; les n-, iso-, néo- et cycloalcanes non-, partiellement et perfluorés ; les mélanges phosphate d'hydrogène/carbonate d'hydrogène/amidon ; le nitrite d'ammonium ; le carbonate de calcium ; le carbonate d'ammonium ; les mélanges de carbonate et d'acides solides ; les monomères, oligomères et polymères éliminant facilement les gaz, en particulier l'acide maléique ainsi que ses esters et anhydrides, les acides oxo-carboxyliques, l'acide acéto acétique et ses dérivés, l'acide á-cétopropionique, l'acide acétondicarboxylique, l'acide tartrique ainsi que les esters et sels de celui-ci, les oxalates, les analogues des produits d'addition de Diels-Alder obtenus à partir de diènes avec du dioxyde de carbone ou de l'azote, la 3,6-dihydro-2H-pyran-2-one [26677-08-7], les homo et copolymères de l'acide itaconique et des esters de celui-ci, les copolymères de l'acide azobisisobutyrique avec les diols, l'éthylène glycol, le diéthylène glycol, le triéthylène glycol, le tétraéthylène glycol, l'oligoéthylène glycol, le polyéthylène glycol, le propanediol, le butanediol, l'hexanediol ; les composés azo, en particulier l'azodicarbonamide et l'azodicarbonamide modifié ; les dérivés de l'hydrazine, en particulier le 4,4'-oxybis[benzol]-sulfonhydrazide, le diphénylsulfon-3,3' -di-sulfonhydrazide, le diphénylène, oxyde-4,4-disulfonhydrazide, la trihydrazinotriazine ; les semicarbazides, en particulier le p-toluylène-sulfonyl semicarbazide ; les tétrazoles, en particulier le 5-phényltétrazole ; les benzooxazines, en particulier l'anhydride d'acide isatoïque.

34. Procédé de production d'un implant selon la revendication 1, **caractérisé en ce que**, pendant la préparation- des éléments détectables par ultrasons, une mousse à pores ouverts est gazée puis fermée par un film appliqué thermiquement en surface, des gaz avec une solubilité médiocre dans le sang et les polymères étant de préférence utilisés, en particulier au moins l'un des gaz choisis dans le groupe suivant : le perfluorométhane, le perfluoroéthane, les perfluoropropanes, les perfluorobutanes, les perfluoropentanes.

35. Procédé de production d'un implant selon la revendication 1, **caractérisé en ce que**, pendant la préparation des éléments détectables par ultrasons, une mousse à pores ouverts est gazée puis scellée par revêtement avec une poudre douce biocompatible, la poudre contenant de préférence au moins l'une des substances choisies dans le groupe suivant : des graisses, des cires, des polymères fondant facilement, puis un procédé d'application thermique de film étant de préférence réalisé.

36. Procédé de production d'un implant selon la revendication 1, **caractérisé en ce que**, pendant la préparation des éléments détectables par ultrasons, une mousse à pores ouverts est gazée et scellée avec un matériau de revêtement dissous dans un solvant, le solvant étant alors évaporé.

37. Procédé de production d'un implant selon la revendication 1, **caractérisé en ce que**, pendant la fabrication des éléments détectables par ultrasons, une mousse à pores ouverts est gazée puis scellée avec un revêtement constitué d'un monomère, le revêtement étant alors polymérisé ou réticulé.

38. Procédé de production d'un implant selon la revendication 1, **caractérisé en ce que**, pendant la préparation des éléments détectables par ultrasons, une mousse syntactique est extrudée, faite de préférence de polypropylène, des boules creuses en verre remplies de gaz étant de préférence incluses dans la mousse syntactique.

39. Procédé de production d'un implant selon la revendication 1, **caractérisé en ce que**, pendant la préparation des éléments détectables par ultrasons, un polymère est précipité à partir d'un solvant en présence de microcapsules contenant des gaz ou produisant des gaz.

40. Procédé de production d'un implant selon la revendication 1, **caractérisé, en ce que**, pendant la préparation des éléments détectables par ultrasons, une polymérisation interfaciale avec des microcapsules contenant des gaz ou produisant des gaz est réalisée.

41. Procédé de production d'un implant selon la revendication 1, **caractérisé en ce que**, pendant la préparation des éléments détectables par ultrasons, en présence de microcapsules contenant des gaz ou produisant des gaz une polymérisation ou une polyaddition ou une polycondensation d'au moins un monomère ou polymère hydrophile et d'un agent de réticulation chimique est réalisée.

42. Procédé selon la revendication 41, **caractérisé en ce que** l'hydrogel obtenu par polymérisation ou polyaddition ou polycondensation contient au moins l'une des substances choisies dans le groupe suivant : le méthacrylate d'hydroxy-éthyle (HEMA) polymérisé ; le méthacrylate d'hydroxypropyle (HPMA) polymérisé ; l'α-méthacryloyl-ω-méthoxy polyéthylène glycol polymérisé ; le polyéthylène glycol-bisacrylate polymérisé ; les prépolymères résorbables de type A-B-C-B-A avec A = groupes acryle ou méthacryle, B = groupes pouvant être divisés hydrolytiquement et contenant des polymères de lactide, de glycolide, d'acide 2-hydroxy-butyrique, d'acide 2-hydroxyvalérique, de carbonate de triméthylène, de polyorthoesters, de polyanhydrides, de polyphosphates, de polyphosphazènes et/ou de polyamides et/ou de copolymères de ceux-ci, et C = polymères hydrophiles, en particulier le polyéthylène glycol (PEG), le polyvinyl alcool (PVA), la polyvinyl pyrrolidone (PVP), le poly-N-isoprolyacrylamide (PNiPAAM).

43. Procédé de production d'un implant selon la revendication 1, **caractérisé en ce que**, pendant la préparation des éléments détectables par ultrasons, un fil creux ou un tube fin est scellé thermiquement au moins aux deux extrémités.

44. Procédé de production d'un implant selon la revendication 1, **caractérisé en ce que**, pendant la préparation des éléments détectables par ultrasons, un fil creux ou un tube fin est scellé par des ultrasons au moins aux deux extrémités puis de préférence revêtu d'un matériau d'étanchéité fait de cire et/ou d'un polymère.

45. Procédé selon la revendication 43 ou 44, **caractérisé en ce que**, avant le scellement, un agent est versé dans le fil creux ou le tube fin qui libère un gaz après l'introduction d'humidité, l'agent contenant de préférence l'une des substances suivantes : levure chimique, mélanges acide benzoïque/carbonate, carbures, hydrures métalliques.

46. Procédé selon la revendication 43 ou 44, **caractérisé en ce que**, avant le scellement, une suspension de microcapsules contenant des gaz ou produisant des gaz est versée dans le fil creux ou le tube fin.

47. Procédé selon la revendication 46, **caractérisé en ce que** des inhibiteurs de décomposition pour les microcapsules sont de plus versés dans le fil creux ou le tube fin avant le scellement, les inhibiteurs de décomposition contenant de préférence au moins l'une des substances suivantes : des tampons, des acides, des bases, des polymères qui ajustent la valeur du pH par décomposition ou solvatation, des inhibiteurs d'estérase, des inhibiteurs de protéase, des inhibiteurs de dextranase, des inhibiteurs d'oxydase à fonction mixte, des cryoprotecteurs ou des enzymes qui favorisent la décomposition des enzymes qui décomposent les microcapsules, des polymères décomposables de manière compétitive non détectables par ultrasons.

48. Procédé selon la revendication 43 ou 44, **caractérisé en ce que**, avant le scellement, une suspension de microcapsules contenant des gaz ou produisant des gaz sous une forme sèche est versée dans le fil creux ou le tube fin.

49. Procédé selon la revendication 43 ou 44, **caractérisé en ce que**, avant le scellement, une émulsion qui s'évapore sous les ultrasons utilisés pour le diagnostic est versée dans le fil creux ou le tube fin.

50. Procédé de production d'un implant selon la revendication 1, **caractérisé en ce que**, pour la préparation en douceur des éléments détectables par ultrasons, des microparticules remplies de gaz sont encapsulées dans un hydrogel au-dessous de 50 °C en présence de monomères et/ou polymères hydrophiles mono- ou bifonctionnels.

51. Procédé de production d'un implant selon la revendication 1, **caractérisé en ce que**, pour la préparation en douceur des éléments détectables par ultrasons, des microparticules remplies de gaz sont encapsulées au-dessous de 50 °C en présence d'un dialdéhyde et d'une polyamine ou d'une protéine.

52. Procédé selon la revendication 50 ou 51, **caractérisé en ce que** l'encapsulation est réalisée directement sur l'implant.

53. Procédé selon la revendication 50 ou 51, **caractérisé en ce que** l'encapsulation est réalisée sous la forme de corps pré-formés ou de structures linéaires, en particulier des fils, et les corps pré-formés ou les structures linéaires sont attachés à la structure de base flexible de l'implant après l'encapsulation.

54. Procédé de production d'un implant selon la revendication 1, **caractérisé en ce que**, pendant la préparation des éléments détectables par ultrasons, des microcapsules remplies de gaz ou leurs suspensions sont utilisées comme étapes préliminaires à la génération in situ de bulles dans les corps pré-formés ou les structures linéaires, en particulier des fils.

55. Procédé de production d'un implant selon la revendication 1, **caractérisé en ce que** des microcapsules échogènes sont utilisées comme précurseurs pour générer des bulles dans l'implant.
